(19) Europäisches Patentamt
European Patent Office
Office européen des brevets

(11) EP 3 636 760 A1

(12) EUROPEAN PATENT APPLICATION
published in accordance with Art. 153(4) EPC

(43) Date of publication:
**15.04.2020 Bulletin 2020/16**

(21) Application number: **18814269.9**

(22) Date of filing: **24.05.2018**

(51) Int Cl.:
***C12N 15/113*** *(2010.01)* ***C12N 15/63*** *(2006.01)*
***C12N 9/22*** *(2006.01)*

(86) International application number:
**PCT/CN2018/088105**

(87) International publication number:
**WO 2018/223843 (13.12.2018 Gazette 2018/50)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.06.2017 CN 201710413147**

(71) Applicant: **Guangzhou Ribobio Co., Ltd.**
**Guangzhou 510663 (CN)**

(72) Inventor: **ZHANG, Biliang**
**Guangzhou**
**Guangdong 510663 (CN)**

(74) Representative: **Biggi, Cristina et al**
**Bugnion S.p.A.**
**Viale Lancetti 17**
**20158 Milano (IT)**

# (54) SYSTEM FOR DNA EDITING AND APPLICATION THEREOF

(57) Provided is a system for DNA editing, comprising crRNA and tracrRNA. The crRNA is an RNA shown in formula I: Nx-ncrRNA (formula I), wherein the Nx is a spacer, and the ncrRNA is an RNA shown in SEQ ID NOs. 1-4 of the Sequence Listing. The tracrRNA is an RNA shown in SEQ ID NOs. 5-8 of the Sequence Listing.

EP 3 636 760 A1

SEQUENCE LISTING

<110> GUANGZHOU RIBOBIO CO., LTD.

<120> SYSTEM FOR DNA EDITING AND APPLICATION THEREOF

<130> PN114630RBSW

<150> PCT/CN2018/088105
<151> 2018-05-24

<160> 29

<170> PatentIn version 3.5

<210> 1
<211> 12
<212> RNA
<213> Artificial Sequence

<220>
<223> ncrRNA

<400> 1
guuuuagagc ua 12

<210> 2
<211> 14
<212> RNA
<213> Artificial Sequence

<220>
<223> 12-14nt RNA

<400> 2
guuuuagagc uaug 14

<210> 3
<211> 16
<212> RNA
<213> Artificial Sequence

<220>
<223> 12-16nt RNA

<400> 3
guuuuagagc uaugcu 16

<210> 4
<211> 18
<212> RNA
<213> Artificial Sequence

<220>
<223> 12-18nt RNA

<400> 4
guuuuagagc uaugcugu 18

<210> 5
<211> 64
<212> RNA
<213> Artificial Sequence

<220>
<223> tracrRNA1

<400> 5
uagcaaguua aaauaaggcu aguccguuau caacuugaaa aaguggcacc gagucggugc 60

uuuu 64

<210> 6
<211> 66
<212> RNA
<213> Artificial Sequence

<220>
<223> tracrRNA2

<400> 6
aauagcaagu uaaaauaagg cuaguccguu aucaacuuga aaaaguggca ccgagucggu 60

gcuuuu 66

<210> 7
<211> 68
<212> RNA
<213> Artificial Sequence

<220>
<223> tracrRNA3

<400> 7
gaaauagcaa guuaaaauaa ggcuaguccg uuaucaacuu gaaaaagugg caccgagucg 60

gugcuuuu 68

<210> 8
<211> 70
<212> RNA
<213> Artificial Sequence

<220>
<223> tracrRNA4

<400> 8
uagaaauagc aaguuaaaau aaggcuaguc cguuaucaac uugaaaaagu ggcaccgagu 60

cggugcuuuu 70

<210> 9
<211> 32
<212> RNA
<213> Artificial Sequence

<220>
<223> RNA-2

<400> 9
ccacagggaa gcugggugaa guuuuagagc ua 32

<210> 10
<211> 34
<212> RNA
<213> Artificial Sequence

<220>
<223> RNA-2

<400> 10
ccacagggaa gcugggugaa guuuuagagc uaug 34

<210> 11
<211> 36
<212> RNA
<213> Artificial Sequence

<220>
<223> RNA-2

<400> 11
ccacagggaa gcugggugaa guuuuagagc uaugcu 36

<210> 12
<211> 38
<212> RNA
<213> Artificial Sequence

<220>
<223> RNA-2

<400> 12
ccacagggaa gcugggugaa guuuuagagc uaugcugu 38

<210> 13
<211> 34
<212> RNA
<213> Artificial Sequence

<220>
<223> RNA-2 while the DNA is the Oct4 gene

<400> 13
gguuauuucu agaaguuagg guuuuagagc uaug 34

<210> 14
<211> 34
<212> RNA
<213> Artificial Sequence

<220>
<223> the RNA-2 while the DNA is the Oct4 gene,

<400> 14
cuucuacaga cuauuccuug guuuuagagc uaug 34

<210> 15
<211> 34
<212> RNA
<213> Artificial Sequence

<220>
<223> the RNA-2 while the DNA is the Oct4 gene,

<400> 15
ggaaagggga gauugauaac guuuuagagc uaug 34

<210> 16
<211> 34
<212> RNA
<213> Artificial Sequence

<220>
<223> the RNA-2 while the DNA is the EMX1 gene

<400> 16
aaggccgugc ggauccgcuu guuuuagagc uaug 34

<210> 17
<211> 34
<212> RNA
<213> Artificial Sequence

<220>
<223> the RNA-2 while the DNA is the EMX1 gene

<400> 17
agguccgacg uguuggagug guuuuagagc uaug 34

<210> 18
<211> 34
<212> RNA
<213> Artificial Sequence

<220>
<223> ,the RNA-2 while the DNA is the EMX1 gene

<400> 18
cgaugucacc uccaaugacu guuuuagagc uaug 34

<210> 19
<211> 34
<212> RNA
<213> Artificial Sequence

<220>
<223> the RNA-2 while the DNA is the EMX1 gene

<400> 19
ucucgcccuc gcagcugcug guuuuagagc uaug 34

<210> 20

<211> 34
<212> RNA
<213> Artificial Sequence

<220>
<223> the RNA-2 while the DNA is the ¦Â thalassemia gene

<400> 20
guguggcaaa ggugcccuug guuuuagagc uaug 34

<210> 21
<211> 34
<212> RNA
<213> Artificial Sequence

<220>
<223> the RNA-2 while the DNA is the ¦Â thalassemia gene

<400> 21
accaauagaa acugggcaug guuuuagagc uaug 34

<210> 22
<211> 34
<212> RNA
<213> Artificial Sequence

<220>
<223> the RNA-2 while the DNA is the ¦Â thalassemia gene

<400> 22
cguaaauaca cuugcaaagg guuuuagagc uaug 34

<210> 23
<211> 34
<212> RNA
<213> Artificial Sequence

<220>
<223> the RNA-2 while the DNA is the TP53 gene,

<400> 23
caauucugcc cucacagcuc guuuuagagc uaug 34

<210> 24
<211> 34
<212> RNA
<213> Artificial Sequence

<220>
<223> the RNA-2 while the DNA is the TP53 gene

<400> 24
ccccaaaaug uuaguaucua guuuuagagc uaug 34

<210> 25
<211> 34
<212> RNA
<213> Artificial Sequence

<220>
<223>  the RNA-2  while the DNA is the TP53 gene

<400>  25
cccucccaug ugcucaagac guuuuagagc uaug    34

<210>  26
<211>  34
<212>  RNA
<213>  Artificial Sequence

<220>
<223>  the RNA-2  while the DNA is the TP53 gene

<400>  26
ugggagcgug cuuuccacga guuuuagagc uaug    34

<210>  27
<211>  34
<212>  RNA
<213>  Artificial Sequence

<220>
<223>  the RNA-2  while the DNA is the TP53 gene

<400>  27
ccagucuuga gcacauggga guuuuagagc uaug    34

<210>  28
<211>  34
<212>  RNA
<213>  Artificial Sequence

<220>
<223>  the RNA-2 while the DNA is the VEGFA gene

<400>  28
ugacugccgu cugcacaccc guuuuagagc uaug    34

<210>  29
<211>  34
<212>  RNA
<213>  Artificial Sequence

<220>
<223>  the RNA-2 while the DNA is the VEGFA gene

<400>  29
aagaggagag ggggccgcag guuuuagagc uaug    34

SEQUENCE LISTING

<110>  GUANGZHOU RIBOBIO CO., LTD.

<120>  SYSTEM FOR DNA EDITING AND APPLICATION THEREOF

<130>  PN114630RBSW

<150>  PCT/CN2018/088105
<151>  2018-05-24

<160>  66

<170>  PatentIn version 3.5

<210>  1
<211>  12
<212>  RNA
<213>  Artificial Sequence

<220>
<223>  ncrRNA

<400>  1
guuuuagagc ua    12

<210>  2
<211>  14
<212>  RNA
<213>  Artificial Sequence

<220>
<223>  12-14nt RNA

<400>  2
guuuuagagc uaug    14

<210>  3
<211>  16
<212>  RNA
<213>  Artificial Sequence

<220>
<223>  12-16nt RNA

<400>  3
guuuuagagc uaugcu    16

<210>  4
<211>  18
<212>  RNA
<213>  Artificial Sequence

<220>
<223>  12-18nt RNA

<400>  4
guuuuagagc uaugcugu    18

<210> 5
<211> 64
<212> RNA
<213> Artificial Sequence

<220>
<223> tracrRNA1

<400> 5
uagcaaguua aaauaaggcu aguccguuau caacuugaaa aaguggcacc gagucggugc 60

uuuu 64

<210> 6
<211> 66
<212> RNA
<213> Artificial Sequence

<220>
<223> tracrRNA2

<400> 6
aauagcaagu uaaaauaagg cuaguccguu aucaacuuga aaaaguggca ccgagucggu 60

gcuuuu 66

<210> 7
<211> 68
<212> RNA
<213> Artificial Sequence

<220>
<223> tracrRNA3

<400> 7
gaaauagcaa guuaaaauaa ggcuaguccg uuaucaacuu gaaaaagugg caccgagucg 60

gugcuuuu 68

<210> 8
<211> 70
<212> RNA
<213> Artificial Sequence

<220>
<223> tracrRNA4

<400> 8
uagaaauagc aaguuaaaau aaggcuaguc cguuaucaac uugaaaaagu ggcaccgagu 60

cggugcuuuu 70

<210> 9
<211> 32
<212> RNA
<213> Artificial Sequence

<220>
<223> RNA-2

<400> 9
ccacagggaa gcugggugaa guuuuagagc ua 32

<210> 10
<211> 34
<212> RNA
<213> Artificial Sequence

<220>
<223> RNA-2

<400> 10
ccacagggaa gcugggugaa guuuuagagc uaug 34

<210> 11
<211> 36
<212> RNA
<213> Artificial Sequence

<220>
<223> RNA-2

<400> 11
ccacagggaa gcugggugaa guuuuagagc uaugcu 36

<210> 12
<211> 38
<212> RNA
<213> Artificial Sequence

<220>
<223> RNA-2

<400> 12
ccacagggaa gcugggugaa guuuuagagc uaugcugu 38

<210> 13
<211> 34
<212> RNA
<213> Artificial Sequence

<220>
<223> RNA-2 while the DNA is the Oct4 gene

<400> 13
gguuauuucu agaaguuagg guuuuagagc uaug 34

<210> 14
<211> 34
<212> RNA
<213> Artificial Sequence

<220>
<223> the RNA-2 while the DNA is the Oct4 gene,

<400> 14
cuucuacaga cuauuccuug guuuuagagc uaug 34

<210> 15
<211> 34
<212> RNA
<213> Artificial Sequence

<220>
<223> the RNA-2 while the DNA is the Oct4 gene,

<400> 15
ggaaagggga gauugauaac guuuuagagc uaug 34

<210> 16
<211> 34
<212> RNA
<213> Artificial Sequence

<220>
<223> the RNA-2 while the DNA is the EMX1 gene

<400> 16
aaggccgugc ggauccgcuu guuuuagagc uaug 34

<210> 17
<211> 34
<212> RNA
<213> Artificial Sequence

<220>
<223> the RNA-2 while the DNA is the EMX1 gene

<400> 17
agguccgacg uguuggagug guuuuagagc uaug 34

<210> 18
<211> 34
<212> RNA
<213> Artificial Sequence

<220>
<223> ,the RNA-2 while the DNA is the EMX1 gene

<400> 18
cgaugucacc uccaaugacu guuuuagagc uaug 34

<210> 19
<211> 34
<212> RNA
<213> Artificial Sequence

<220>
<223> the RNA-2 while the DNA is the EMX1 gene

<400> 19
ucucgcccuc gcagcugcug guuuuagagc uaug 34

<210> 20

<211> 34
<212> RNA
<213> Artificial Sequence

<220>
<223> the RNA-2 while the DNA is the thalassemia gene

<400> 20
guguggcaaa ggugcccuug guuuuagagc uaug 34

<210> 21
<211> 34
<212> RNA
<213> Artificial Sequence

<220>
<223> the RNA-2 while the DNA is the thalassemia gene

<400> 21
accaauagaa acugggcaug guuuuagagc uaug 34

<210> 22
<211> 34
<212> RNA
<213> Artificial Sequence

<220>
<223> the RNA-2 while the DNA is the thalassemia gene

<400> 22
cguaaauaca cuugcaaagg guuuuagagc uaug 34

<210> 23
<211> 34
<212> RNA
<213> Artificial Sequence

<220>
<223> the RNA-2 while the DNA is the TP53 gene,

<400> 23
caauucugcc cucacagcuc guuuuagagc uaug 34

<210> 24
<211> 34
<212> RNA
<213> Artificial Sequence

<220>
<223> the RNA-2 while the DNA is the TP53 gene

<400> 24
ccccaaaaug uuaguaucua guuuuagagc uaug 34

<210> 25
<211> 34
<212> RNA
<213> Artificial Sequence

<220>
<223> the RNA-2 while the DNA is the TP53 gene

<400> 25
cccucccaug ugcucaagac guuuuagagc uaug 34

<210> 26
<211> 34
<212> RNA
<213> Artificial Sequence

<220>
<223> the RNA-2 while the DNA is the TP53 gene

<400> 26
ugggagcgug cuuuccacga guuuuagagc uaug 34

<210> 27
<211> 34
<212> RNA
<213> Artificial Sequence

<220>
<223> the RNA-2 while the DNA is the TP53 gene

<400> 27
ccagucuuga gcacauggga guuuuagagc uaug 34

<210> 28
<211> 34
<212> RNA
<213> Artificial Sequence

<220>
<223> the RNA-2 while the DNA is the VEGFA gene

<400> 28
ugacugccgu cugcacaccc guuuuagagc uaug 34

<210> 29
<211> 34
<212> RNA
<213> Artificial Sequence

<220>
<223> the RNA-2 while the DNA is the VEGFA gene

<400> 29
aagaggagag gggccgcag guuuuagagc uaug 34

<210> 30
<211> 20
<212> DNA
<213> Homo sapiens

<400> 30
tgccgctcac tttgatgtct 20

<210> 31
<211> 17
<212> DNA
<213> Homo sapiens

<400> 31
gagcctcagc ccctcca 17

<210> 32
<211> 20
<212> DNA
<213> Homo sapiens

<400> 32
atggcacatt gtcagaggga 20

<210> 33
<211> 21
<212> DNA
<213> Homo sapiens

<400> 33
gggagcagga aagtgaggtt a 21

<210> 34
<211> 20
<212> DNA
<213> Mus musculus

<400> 34
ggtcagagag agcgcgcggg 20

<210> 35
<211> 21
<212> DNA
<213> Mus musculus

<400> 35
gggcacgacc gcttaccttg g 21

<210> 36
<211> 23
<212> DNA
<213> Mus musculus

<400> 36
gaccagtcgc gctgacggac aga 23

<210> 37
<211> 19
<212> DNA
<213> Mus musculus

<400> 37
cgcggctcgc gctccctct 19

<210> 38
<211> 24
<212> DNA
<213> Homo sapiens

<400> 38
cttctgagac atgatgctct tcct 24

<210> 39
<211> 20
<212> DNA
<213> Homo sapiens

<400> 39
tcatgggtga gggtagtctg 20

<210> 40
<211> 20
<212> DNA
<213> Homo sapiens

<400> 40
catcccttgg atgtgccagt 20

<210> 41
<211> 20
<212> DNA
<213> Homo sapiens

<400> 41
ttagagggga gatgcggtca 20

<210> 42
<211> 21
<212> DNA
<213> Homo sapiens

<400> 42
tgcgtgtcaa ggaatggaga g 21

<210> 43
<211> 22
<212> DNA
<213> Homo sapiens

<400> 43
gacagccttt ccagtgattc ag 22

<210> 44
<211> 22
<212> DNA
<213> Homo sapiens

<400> 44
aggctctaaa gacgggcttg ac 22

<210> 45
<211> 20
<212> DNA
<213> Homo sapiens

<400> 45
tcaggaggcc caacccagat 20

<210> 46
<211> 20
<212> DNA
<213> Homo sapiens

<400> 46
acacgcagaa gcagcctgac 20

<210> 47
<211> 20
<212> DNA
<213> Homo sapiens

<400> 47
ctgggcctgg tgggaaatag 20

<210> 48
<211> 20
<212> DNA
<213> Homo sapiens

<400> 48
gtaaggtccg ccgaagaaag 20

<210> 49
<211> 18
<212> DNA
<213> Homo sapiens

<400> 49
tttcggagca gtcgagtg 18

<210> 50
<211> 19
<212> DNA
<213> Homo sapiens

<400> 50
gtctgaagct gcgaccttg 19

<210> 51
<211> 21
<212> DNA
<213> Homo sapiens

<400> 51
ctggctttgc tgctgttcct g 21

<210> 52
<211> 23
<212> DNA
<213> Homo sapiens

<400> 52
gcagttagaa cagcccagct agt 23

<210> 53
<211> 21
<212> DNA
<213> Homo sapiens

<400> 53
ctgagacttc cacactgatg c 21

<210> 54
<211> 21
<212> DNA
<213> Homo sapiens

<400> 54
gcatattctg gagacgcagg a 21

<210> 55
<211> 22
<212> DNA
<213> Homo sapiens

<400> 55
tggtgtctgt ttgaggttgc ta 22

<210> 56
<211> 23
<212> DNA
<213> Homo sapiens

<400> 56
catattctgg agacgcagga aga 23

<210> 57
<211> 21
<212> DNA
<213> Homo sapiens

<400> 57
aaacatcaag cgtcccatag a 21

<210> 58
<211> 22
<212> DNA
<213> Homo sapiens

<400> 58
actgtgttca ctagcaacct ca 22

<210> 59
<211> 22
<212> DNA
<213> Homo sapiens

<400> 59
gggtgtgata ttacggaaag cc 22

<210> 60
<211> 20
<212> DNA
<213> Homo sapiens

<400> 60
tacacccagg tctcccaaca 20

<210> 61
<211> 20
<212> DNA
<213> Homo sapiens

<400> 61
ccagctgaga gcaaacgcaa 20

<210> 62
<211> 20
<212> DNA
<213> Homo sapiens

<400> 62
aatacacgga gccgagagcc 20

<210> 63
<211> 20
<212> RNA
<213> Artificial Sequence

<220>
<223> Nx sequence of a crRNA

<400> 63
ccacagggaa gcugggugaa 20

<210> 64
<211> 34
<212> RNA
<213> Artificial Sequence

<220>
<223> 2′-O-methyl modification on the 2nd, 30th, 31st and 33rd sites of the crRNA5

<400> 64
ugacugccgu cugcacaccc guuuuagagc uaug 34

**<210> 65**
**<211> 34**
**<212> RNA**
**<213> Artificial Sequence**

**<220>**
**<223> cholesterol modification on crRNA2**

**<400> 65**
**ugacugccgu cugcacaccc guuuuagagc uaug 34**

**<210> 66**
**<211> 34**
**<212> RNA**
**<213> Artificial Sequence**

**<220>**
**<223> 2'-O-methyl modification on the 2nd,30th, 31st and 33rd sites of the VEGFA-crRNA6**

**<400> 66**
**aagaggagag ggggccgcag guuuuagagc uaug 34**

## Description

### Technical Field

The present invention relates to a system for DNA editing and uses thereof in the field of biotechnologies.

### Background

A CRISPR-Cas system is an adaptive immunity defense mechanism formed in a long-term evolutionary process of bacteria and archaebacteria, and may be used for resisting invasive viruses and an exogenous DNA. According to different functional elements, the CRISPR-Cas system may be sorted as a system I, a system II and a system III. The three types of the systems may be sorted as more subclasses according to different genes encoding Cas proteins thereof. The system I and the system III of the CRISPR-Cas system only require participation of two elements of crRNA and Cas protein, but the CRISPR-Cas system II includes three elements of crRNA, tracrRNA and the Cas protein.
A CRISPR/Cas9 system, through integrating fragments of invasive phages and a plasmid DNA into CRISPR, and using corresponding CRISPR RNAs(crRNAs) to guide the degradation of homologous sequences, thereby provides immunity. The working principle of the system is that CRISPR-derived RNA (crRNA) is combined with trans-activating RNA (tracrRNA) through base pairing to form a tracrRNA/crRNA compound, and the compound guides nuclease Cas9 protein to shear the double-strand DNA at a target site paired with the crRNA.
Cas9 has two key structural domains: HNH and RuvC, they respectively digest a single strand of the double-strand DNA. DNA double strand break (DSB) is generated, a cell starts a repair mechanism, and the cell may generate gene site-directed mutation through an inaccurate repair mode, such as non-homologous end joining (NHEJ), or realize accurate gene site-directed insertion or gene replacement through repair of a homologous recombination (HR) mode. The Cas9 has already been successfully performed genome engineering research in bacteria, human cells, zebra fish and mice.

### Summary

The present invention aims to provide a CRISPR/Cas system for DNA editing.
The present invention firstly provides a system for DNA editing, the system is the CRISPR/Cas system, including A or B:
A, the following N1) and N2):

N1) is a RNA named as RNA-2 or a biological material related to the RNA-2;
N2) is a RNA named as RNA-1 or a biological material related to the RNA-1;

The RNA-2 is the RNA as shown in a formula I:

Nx-ncrRNA

The formula I:
wherein, the Nx is a spacer in the CRISPR/Cas system;
The ncrRNA is any one of the following a1) to a4):

a1) a RNA as shown in SEQ ID NO.1 of the sequence listing;
a2) a RNA acquired by adding one or more nucleotides to 5'-terminal and/or 3'-terminal of the a1);
a3) a RNA having more than 85% of identity as the RNA defined in a1) or a2); and
a4) a modifier of the RNA as shown in SEQ ID NO.1 of the sequence listing.

The biological material related to the RNA-2 is any one of the following A1) to A5):

A1) a DNA molecule for encoding the RNA-2;
A2) an expression cassette containing the DNA molecule of A1);
A3) a recombinant vector containing the DNA molecule of A1), or a recombinant vector containing the expression cassette of the A2);
A4) a recombinant microorganism containing the DNA molecule of A1), or a recombinant microorganism containing the expression cassette of A2), or a recombinant microorganism containing the recombinant vector of A3); and
A5) a cell line containing the DNA molecule of A1), or a cell line containing the expression cassette of A2).

The RNA-1 is any one of the following b1) to b4):

b1) a RNA as shown in SEQ ID NO.5 of the sequence listing;
b2) a RNA acquired by adding one or more nucleotides to 5'-terminal and/or 3'-terminal of the b1);
b3) a RNA having more than 85% of identity as the RNA defined in b1) or b2); and
b4) a modifier of the RNA as shown in SEQ ID NO.5 of the sequence listing.

The biological material related to the RNA-1 is any one of the following B1) to B5):

B1) a DNA molecule for encoding the RNA-1;
B2) an expression cassette containing the DNA molecule of B1);
B3) a recombinant vector containing the DNA molecule of B1), or a recombinant vector containing the expression cassette of B2);
B4) a recombinant microorganism containing the DNA molecule of B1), or a recombinant microorganism containing the expression cassette of B2), or a recombinant microorganism containing the recombinant vector of B3); and
B5) a cell line containing the DNA molecule of B1), or a cell line containing the expression cassette of B2).

The ncrRNA and a partial fragment of the RNA-1 are complemented to form a structure containing a stem area and a ring area.
B, the RNA-2.
The Nx part of the RNA-2 is positioned at the 5'-terminal, the ncrRNA part of the RNA-2 is positioned at the 3'-terminal, the two parts are connected through a bond between the nucleosides. Nx is the RNA sequence, and complemented with a fragment of a target DNA for editing, and a requirement for the spacer in the CRISPR/Cas system is satisfied.
N may be a ribonucleotide or a ribonucleotide modifier; x is a number of the N.
Wherein, the x is a non-zero natural number. The x specifically may be any one of the following e1)-e3):

e1) any one integer between 15 and 22;
e2) any one integer between 19 and 21;
e3) 20.

The RNA-2 and the partial fragment of the RNA-1 form a compound through the ncrRNA, a Cas protein (namely Cas nuclease) in the CRISPR-Cas system may be combined with the compound to realize the editing of the DNA.
The step of adding one or more nucleotides is specifically a step of adding one to ten nucleotides.
A term 'identity' used herein is sequence similarity to a natural nucleotide sequence. The 'identity' includes the nucleotide sequences having 85% or more, or 90% or more, or 95% or more of the identity with the nucleotide sequence as shown in the SEQ ID NO.1 or the SEQ ID NO.5 of the present invention. The identity may be evaluated with naked eyes or computer software. When the computer software is used, the identity between two or more sequences may be expressed by a percentage (%), the percentage may be used for evaluating the identity between the related sequences.
The rigorous condition is as follows: in solution of 2×SSC, 0.1%SDS, hybridizing in 68 DEG C and membrane-washing for twice, 5 min each time, and in solution of 0.5×SSC, 0.1%SDS, hybridizing in 68 DEG C and membrane-washing for twice, 15 min each time; or, in solution of 0.1 xSSPE(or 0.1×SSC), 0.1%SDS, hybridizing in 65 DEG C and membrane-washing.
The above more than 85% of the identity may be more than 85%, 90% or 95% of the identity.
Those of ordinary skill in the art may easily perform mutation on SEQ ID NO.1 or SEQ ID NO.5 of the present invention with a known method, for example methods of directed evolution and point mutation. Those artificially modified nucleotides which have 75% or more of the identity with the nucleotide sequences of the SEQ ID NO.1 or SEQ ID NO.5 of the present invention are derived from the nucleotide sequences of the present invention and equivalent to the sequence of the present invention only if the nucleotides have the same function.
The cell line does not include a propagation material.
In the above system, the RNA of the a2) may be any one of the following a21) to a23):

a21) a 12-14nt RNA;
a22) a 12-16nt RNA; and
a23) a 12-18nt RNA.

The RNA of the b2) may be any one of the following b21) to b24):

b21) a 64-66nt RNA;

b22) a 64-68nt RNA;
b23) a 64-70nt RNA; and
b24) a 64-86nt RNA.

The ribonucleotide may be A, U, C or G. The ribonucleotide modifier may be a substance acquired by modification A, U, C or G on a ribose, a phosphate skeleton and/or a basic group.
The modifier may be a substance acquired by modification at least one nucleotide in the RNA on the ribose, the phosphate skeleton and/or the basic group.
The modification may be 2'-O-methyl modification, 2'-deoxidation modification, 2'-fluorination modification or cholesterol modification.
The DNA may be any one of the following M1)-M5):

M1) an eukaryote DNA;
M2) an animal DNA;
M3) a mammalian DNA;
M4) a human DNA; and
M5) a mouse DNA.

In the above system, the RNA of the a21) may be the RNA as shown in SEQ ID NO.2 of the sequence listing;
the RNA of a22) may be the RNA as shown in SEQ ID NO.3 of the sequence listing;
the RNA of a23) may be the RNA as shown in SEQ ID NO.4 of the sequence listing;
the RNA of b21) may be the RNA as shown in SEQ ID NO.6 of the sequence listing;
the RNA of b22) may be the RNA as shown in SEQ ID NO.7 of the sequence listing; and
the RNA of b23) may be the RNA as shown in SEQ ID NO.8 of the sequence listing.
The modification may be 2'-O-methyl modification, 2'-deoxidation modification, 2'-fluorination modification or cholesterol modification.
The DNA may be a vascular endothelial growth factor A (VEGFA) gene, an Empty spiracles homeobox 1 (EMX1) gene, an organic cation/carnitine transporter4 (Oct4) gene, a β thalassemia gene, a tumor protein p53 (TP53) gene or a promoter of the TP53 gene.
In the above system, the RNA-2 may be the RNA as show in any one sequence of SEQ ID NO.9-29.
While the DNA is the VEGFA gene, the RNA-2 is the RNA as shown in at least one sequence of SEQ ID NO.9-12, SEQ ID NO.28 and SEQ ID NO.29;
while the DNA is the Oct4 gene, the RNA-2 is the RNA as shown in at least one sequence of SEQ ID NO.13-15;
while the DNA is the EMX1 gene, the RNA-2 is the RNA as shown in at least one sequence of SEQ ID NO.16-19;
while the DNA is the Beta thalassemia gene, the RNA-2 is the RNA as shown in at least one sequence of SEQ ID NO.20-22;
while the DNA is the TP53 gene, the RNA-2 is the RNA as shown in SEQ ID NO.23 and/or 24; and
while the DNA is the promoter of the TP53 gene, the RNA-2 is the RNA as shown in at least one sequence of SEQ ID NO.25-27.
The system may be the CRISPR-Cas system, specifically the CRISPR-Cas system II, for example the CRISPR/Cas9 system.
The above system may further include N3), the N3) is Cas9 nuclease or a biological material related to the Cas9 nuclease.
The biological material related to the Cas9 nuclease is any one of the following C1) to C7):

C1) a nucleic acid molecule for encoding the Cas9 nuclease;
C2) an expression cassette containing the nucleic acid molecule of C1);
C3) a recombinant vector containing the nucleic acid molecule of C1), or a recombinant vector containing the expression cassette of C2);
C4) a recombinant microorganism containing the nucleic acid molecule of C1), or a recombinant microorganism containing the expression cassette of C2), or a recombinant microorganism containing the recombinant vector of C3); and
C5) a cell line containing the nucleic acid molecule of C1), or a cell line containing the expression cassette of C2).

In the above system, the Cas9 nuclease may be derived from R1) or R2):

R1) bacteria; and
R2) streptococcus, staphylococcus, rothia, neisseria, corynebacterium or lactobacillus.

The streptococcus may be *Streptococcus pyogenes* specifically. The staphylococcus may be *staphylococcus aureus* specifically. The Cas9 nuclease may be a protein as shown in Genbank ID: AQM52323.1 specifically.
The system may further includes other reagents and/or instruments required by performing DNA editing through using a CRISPR/Cas9 DNA editing method except the crRNA, the tracrRNA and the Cas9 nuclease.
While the system contains the RNA-2 and the RNA-1, a mole ratio of the RNA-1 and the RNA-2 may be 1:(1-1.5).
While the system contains the Cas9, the tracrRNA and the crRNA, a mole ratio of the Cas9, the tracrRNA and the crRNA may be 1:(1-20):(1:20), specifically may be 1 :(1-8):(1:8), further may be 1:(1-4):(1:4), and more further may be 1:(2-4):(2-4).
While the RNA-2 is at least two RNAs, the RNA-2 in the above mole ratio is calculated by total concentration of multiple RNAs.
Specifically, the system may be the RNA-2 only, or formed by the above N1) and N2), or may be formed by the above N1), N2) and N3).
The present invention further provides the following O1) or O2):

The O1) is the RNA-2 or a biological material related to the RNA-2; and
the O2) is the RNA-1 or a biological material related to the RNA-1.

The present invention further provides any one of the following applications:

X1, an application of the ncrRNA for preparing the crRNA;
X2, an application of the ncrRNA in DNA editing;
X3, an application of the ncrRNA for preparing a DNA editing product;
X4, an application of the RNA-1 as the tracrRNA;
X5, an application of the RNA-1 or the biological material related to the RNA-1 in DNA editing;
X6, an application of the RNA-1 or the biological material related to the RNA-1 for preparing the DNA editing product;
X7, an application of the RNA-2 as the crRNA;
X8, an application of the RNA-2 or the biological material related to the RNA-2 in DNA editing;
X9, an application of the RNA-2 or the biological material related to the RNA-2 for preparing a DNA editing product;
X10, an application of the system in the DNA editing;
X11, an application of the system in interfering gene expression in-vivo or in-vitro; and
X12, an application of the system for establishing animal, plant or cell models of which the DNA is edited.

The product may be the CRISPR-Cas system, specifically may be the CRISPR-Cas system II, for example the CRISPR/Cas9 system.
The present invention further provides a method for editing a DNA. The method includes a step of processing a DNA with the system and realizing editing of the DNA.
In a system for performing the DNA editing with the system, while the system contains the RNA-2, a concentration of the RNA-2 may be 100-800 nM, specifically may be 200-400 nm. While the system contains the RNA-1, a concentration of the RNA-1 may be 100-800 nM, specifically may be 200-400 nm. The CRISPR/Cas9 system may also contain the Cas9 nuclease or the biological material related to the Cas9 nuclease.
While the system contains the Cas9 nuclease, a concentration of the Cas9 nuclease may be 20-400 nM, specifically may be 100-400 nM, further may be 100-200 nM. While the system contains a vector containing a Cas9 nuclease encoding gene, a dosage of the vector may be 1-200μg, further may be 20-100μg, more further may be 30-50μg or 1-5μg. The dosage of the vector is 3μg preferably.
While the CRISPR/Cas9 system is used for editing the gene in the cell, a ratio of the cell and the RNA-2 in the system may be 5000-60000 cells/100 nM RNA-2, further may be 7500-40000 cells/100 nM RNA-2, and more further may be 10000-12500 cells/100 nM RNA-2.
The cell may be the eukaryocyte. The cell further may be a mammalian cell, for example a 293T cell, a HeLa cell, a THP1 cell, a HUVEC cell or a C2C12 mouse myoblast.
The present invention further provides a preparation method for the system. The method includes a step of independently packaging each substance of the system.
The preparation method for the system further may include a step of synthesizing each RNA of the system. The synthesizing may be a synthesizing with a chemical method.
In the present invention, the DNA may be any one of the following M1)-M5):

M1) an eukaryote DNA;
M2) an animal DNA;
M3) a mammalian DNA;

M4) a human DNA; and
M5) a mouse DNA.

Efficiency of the editing is at least 1% or more than 1%, for example 2%, 3%, 5%, 10%, 15%, 20%, 25%, 30%, 40%, 50%, 60%, 70%, 80%, 85% or 90%.

In the present invention, the first site of each sequence is the 5'-terminal of the sequence.

The DNA editing may be the digestion to the DNA.

It is proved from an experiment that the system for the DNA editing of the prevent invention may be used as the CRISPR/Cas9 system for editing the DNA, herein while a length of the ncrRNA part in the RNA-2 as the crRNA is 14 nt (namely the ncrRNA2), Cas9 enzyme digestion efficiency is the highest, in-vitro enzyme digestion efficiency reaches 89.2%, and intracellular enzyme digestion efficiency reaches 29.4%; and while a length of the RNA-1 as the tracrRNA is 66 nt, the Cas9 enzyme digestion efficiency is the optimal, the in-vitro enzyme digestion efficiency reaches 90.7%, and the intracellular enzyme digestion efficiency reaches 29.8%. In addition, after the RNA-2 is modified, the enzyme digestion effect of the Cas9 in the CRISPR/Cas9 system has not an apparent change. While the RNA-2 and the RNA-1 may be used for a CRISPR/Cas9 system element and the CRISPR/Cas9 system is used for editing the target DNA, the editing of the DNA is not affected by the cell and the target DNA, the editing of the DNA can be realized, and the system may also be used for directly editing the DNA in an animal body. While the CRISPR/Cas9 system is used for editing the DNA, editing efficiency may also be improved through using the different RNA-2 of multiple sequences, and DNA editing efficiency may be improved with the cell for stably expressing the Cas9. The RNA-2 and RNA-1 sequences of the present invention are short, and easy to be acquired by chemical synthesis, it is beneficial to improve purity of the RNA and mass production, and preparation and application of the CRISPR/Cas9 system are simplified. In addition, a chemosynthetic RNA element does not require plasmid expression, avoids problems of plasmid construction (compatibility between plasmids, plasmid vector carrying capacity, construction difficulty of multiple plasmid systems and the like), is easier to chemical modification, and suitable for in-vivo application. It is indicated that the RNA-2 and the RNA-1 of the present invention may be used as the crRNA and tracrRNA elements in the CRISPR/Cas9 system for editing the target DNA.

## Brief Description of the Drawings

Fig. 1 is an in-vitro enzyme digestion effect of Cas9 under different crRNAs.
Fig. 2 is an intracellular enzyme digestion effect of the Cas9 under the different crRNAs.
Fig. 3 is an in-vitro enzyme digestion effect of the Cas9 under different tracrRNAs.
Fig. 4 is an intracellular enzyme digestion effect of the Cas9 under the different tracrRNAs.
Fig. 5 is an effect of crRNA modification to the in-vitro enzyme digestion effect of the Cas9.
Fig. 6 is an effect of the crRNA modification to the intracellular enzyme digestion effect of the Cas9.
Fig. 7 is a Cas9 nuclease digestion effect in different cell lines to a VEGFA gene.
Fig. 8 is a Cas9 nuclease digestion effect of different Oct4-E-T1-crRNA concentration. Herein, 800, 400 and 200 are the Oct4-E-T1-crRNA concentration, and units are nM.
Fig. 9 is an in-vitro digestion effect of Cas9 nuclease to an EMX1 gene DNA template under the different crRNAs. Herein, E1R, E7F, Ee3F and E2F respectively represent E1R-crRNA, E7F-crRNA, Ee3F-crRNA and E2F-crRNA.
Fig. 10 is a Cas9 nuclease in-vitro digestion effect of different crRNAs. Herein, Beta-3-T1, Beta-3-T3 and Beta-5-T3 respectively represent Beta-3-T1-crRNA, Beta-3-T3-crRNA and Beta-5-T3-crRNA.
Fig. 11 is an intracellular digestion effect of an Oct4 gene under different crRNAs. Herein, E-T1, E-T2 and E-T3 respectively represent Oct4-E-T1-crRNA, Oct4-E-T2-crRNA and Oct4-E-T3-crRNA.
Fig. 12 is an intracellular digestion effect of the Cas9 nuclease to an EMX1 gene under different Ee3F-crRNA dosages. Herein, 400, 200, 100 and 50 are the dosages of Ee3F-crRNA, and units are ng.
Fig. 13 is an intracellular digestion effect of the Cas9 nuclease to a gene under different crRNAs. Herein, Beta-3-T1, Beta-3-T3 and Beta-5-T3 respectively represent Beta-3-T1-crRNA, Beta-3-T3-crRNA and Beta-5-T3-crRNA.
Fig. 14 is a digestion effect of VEGFA gene in a mouse cell (C2C12) and a mouse in-vivo.
Fig. 15 is an intracellular digestion effect of a promoter and exon region of a TP53 fragment.
Fig. 16 is an in-vitro digestion effect of the promoter and exon region of the TP53 fragment.
Fig. 17 is an in-vitro digestion effect of the Cas9 nuclease to the Oct4 gene under multiple crRNAs. Herein, Mix represents a common experiment result of Oct4-E-T1-crRNA, Oct4-E-T2-crRNA and Oct4-E-T3-crRNA, and ET2 represents Oct4-E-T2-crRNA.
Fig. 18 is an intracellular digestion effect of the Cas9 nuclease to the Oct4 gene under multiple crRNAs. Herein, Mix represents a common experiment result of Oct4-E-T1-crRNA, Oct4-E-T2-crRNA and Oct4-E-T3-crRNA, and ET2 represents Oct4-E-T2-crRNA.
Fig. 19 is an enzyme digestion effect of different Cas9 donors.
Fig. 20 is an enzyme digestion effect of different Cas9 concentration.

## Detailed Description of the Embodiments

The present invention is further described in detail below in combination with specific implementation modes, provided embodiments are only used for illuminating the present invention, but not intended to limit a scope of the present invention. Experiment methods in the following embodiments are conventional methods unless otherwise specified. Materials, reagents, instruments and the like used in the following embodiments may be obtained from a business approach unless otherwise specified. Quantitative tests in the following embodiments set three times of a repeated experiment, and a result is taken from an average value.

Table 1, reagent source

| Name | Source | Article number |
|---|---|---|
| THP-1 cell | ATCC | THP-1 (ATCC® TIB-202<TM>) |
| 293T cell | ATCC | PTA-554 |
| HeLa cell | ATCC | HeLa (ATCC®CCL-2™) |
| HUVEC cell | ATCC | HUVEC (ATCC®CRL-1730™) |
| C2C12 mouse myoblast | ATCC | C2C12 (ATCC® CRL-1772™) |
| DMEM culture medium | GIBCO | C11995 |
| Opti-MEM culture medium | GIBCO | 31985-070 |
| Phusion High-Fidelity DNA Polymerase | Thermo Fisher Scientific | F-530L |
| Blood/tissue/cell genome extracting kit | TIANGEN | DP304 |
| Lipofectamine2000 | Thermo Fisher Scientific | 12566014 |
| TurboFect in-vivo Transfection Reagent | Thermo Scientific | R0541 |
| T7EI endonuclease | NEB | M0302L |
| Cas9 nuclease | NEB | M0386M |
| 10XCas9 buffer | NEB | M0386M |
| pX260 | Addgene | Plasmid:#42229 |

Herein, a sequence of Cas9 nuclease is a sequence as shown in Genbank ID: AQM52323.1 on the NCBI. The protein is derived from *Streptococcus pyogenes*.
A Cas9-293T cell, a Cas9-HeLa cell, a Cas9-THP-1 cell and a Cas9-HUVEC cell in the following embodiments are the cells obtained by respectively importing an encoding gene of Cas9 to the 293T cell, the HeLa cell, the THP-1 cell and the HUVE cell by using a Genome-TALER™& Genome-CRISPR™ human AAVS1 Safe Harbor gene knock-in kit (Catalog# SH-AVS-K100) of the Guangzhou Yijing bio-technology Co., Ltd.

Table 2, primer sequence table

| Primer | sequence(5'-3') |
|---|---|
| VEGFA-F1 | TGCCGCTCACTTTGATGTCT |
| VEGFA-R1 | GAGCCTCAGCCCCTCCA |
| VEGFA-F3 | ATGGCACATTGTCAGAGGGA |
| VEGFA-R3 | GGGAGCAGGAAAGTGAGGTTA |
| VEGFA-outer-F1 (mouse) | GGTCAGAGAGAGCGCGCGGG |
| VEGFA-outer-R1 (mouse) | GGGCACGACCGCTTACCTTGG |

(continued)

| Primer | sequence(5'-3') |
|---|---|
| VEGFA-inner-F1 (mouse) | GACCAGTCGCGCTGACGGACAGA |
| VEGFA-inner-R1 (mouse) | CGCGGCTCGCGCTCCCTCT |
| Oct4-outer-F1 | CTTCTGAGACATGATGCTCTTCCT |
| Oct4-outer-R1 | TCATGGGTGAGGGTAGTCTG |
| Oct4-inner-F1 | CATCCCTTGGATGTGCCAGT |
| Oct4-inner-R1 | TTAGAGGGGAGATGCGGTCA |
| E1-1R/2F-WF/NF | TGCGTGTCAAGGAATGGAGAG |
| E1-1R/2F-WR | GACAGCCTTTCCAGTGATTCAG |
| E1-1R/2F-NR | AGGCTCTAAAGACGGGCTTGAC |
| E1-e3F-NF | TCAGGAGGCCCAACCCAGAT |
| E1-e3F-NR | ACACGCAGAAGCAGCCTGAC |
| E1-7-F-NF | CTGGGCCTGGTGGGAAATAG |
| E1-7-F-NR | GTAAGGTCCGCCGAAGAAAG |
| E1-e3F-WF | TTTCGGAGCAGTCGAGTG |
| E1-e3F-WR | GTCTGAAGCTGCGACCTTG |
| E1-7-F-WF | CTGGCTTTGCTGCTGTTCCTG |
| E1-7-F-WR | GCAGTTAGAACAGCCCAGCTAGT |
| Beta-outer-F1 | CTGAGACTTCCACACTGATGC |
| Beta-outer-R1 | GCATATTCTGGAGACGCAGGA |
| Beta3-inner-F1 | TGGTGTCTGTTTGAGGTTGCTA |
| Beta3-inner-R1 | CATATTCTGGAGACGCAGGAAGA |
| Beta5-inner-F1 | AAACATCAAGCGTCCCATAGA |
| Beta5-inner-R1 | ACTGTGTTCACTAGCAACCTCA |
| TP53-outer-FP | GGGTGTGATATTACGGAAAGCC |
| TP53-outer-RP | TACACCCAGGTCTCCCAACA |
| TP53-inner-FP | CCAGCTGAGAGCAAACGCAA |
| TP53-inner-RP | AATACACGGAGCCGAGAGCC |

Embodiment 1, Preparation of a CRISPR/Cas9 system element for DNA editing

The CRISPR/Cas9 system element for the DNA editing provided by the embodiment is crRNA and tracrRNA.

The crRNA is RNA as shown in a formula I:

Nx-ncrRNA

The formula I:

wherein, N is any one of ribonucleotides and ribonucleotide modifier, the ribonucleotide is any one of A, U, C and G, and the ribonucleotide modifier is a substance obtained by performing 2'-O-methyl modification or cholesterol modification on A, U, C and G;
x is a number of N, and x is 20;
ncrRNA is any one of ncrRNA1, ncrRNA2, ncrRNA2y, ncrRNA3 and ncrRNA4;

ncrRNA1 is a RNA as shown in SEQ ID NO.1 of the sequence listing;
ncrRNA2 is a RNA as shown in SEQ ID NO.2 of the sequence listing;
ncrRNA2y is a substance obtained by respectively performing the 2'-O-methyl modification on the 10-th, 11-th and 13-th nucleotides starting from the 5'-terminal of the ncrRNA2;
ncrRNA 3 is a RNA as shown in SEQ ID NO.3 of the sequence listing;
ncrRNA 4 is a RNA as shown in SEQ ID NO.4 of the sequence listing;
tracrRNA is tracrRNA1, tracrRNA2, tracrRNA3 and tracrRNA4;
tracrRNA1 is a RNA as shown in SEQ ID NO.5 of the sequence listing;
tracrRNA2 is a RNA as shown in SEQ ID NO.6 of the sequence listing;
tracrRNA3 is a RNA as shown in SEQ ID NO.7 of the sequence listing; and
tracrRNA4 is a RNA as shown in SEQ ID NO.8 of the sequence listing.

In the sequence listing, the first site of each sequence is the 5'-terminal of the sequence.

Embodiment 2, Effect of different ncrRNA lengths on DNA editing efficiency

I, In-vitro enzyme digestion detection

1, DNA template preparation

The 293T cell genomic DNA is used as a template, Phusion enzyme (namely Phusion High-Fidelity DNA Polymerase) is used for performing secondary PCR amplification to obtain a vascular endothelial growth factor A (VEGFA) target gene fragment, namely the DNA template. The primer used for the first PCR amplification is VEGFA-F1/VEGFA-R1, the second PCR amplification is performed through using the first PCR amplification product as the template, using VEGFA-F3/VEGFA-R3 as primers, and a length of a second PCR amplification product (namely the DNA template) is 485 bp.

2, Cas9 in-vitro enzyme digestion

Cas9 nuclease, 10×Cas9 buffer, crRNA, tracrRNA and $H_2O$ are uniformly mixed, and incubated at 37 DEG C for 15 min, then the DNA template of the step 1 is added, after uniformly mixed, the incubation is performed for 70 min at 37 DEG C, and the reaction system is specifically as shown in Table 3. The $H_2O$ for replacing crRNA is used as a negative control (neg.). Herein, crRNA is VEGFA-crRNA1, VEGFA-crRNA2, VEGFA-crRNA3 and VEGFA-crRNA4, one crRNA for each reaction system. The structural formula of the VEGFA-crRNA1, the VEGFA-crRNA2, the VEGFA-crRNA3 and the VEGFA-crRNA4 is as shown in the formula I of the embodiment 1, the Nx part is the same, ncrRNA is respectively ncrRNA1, ncrRNA2, ncrRNA3 and ncrRNA4, a sequence of the Nx is 5'-CCACAGGGAAGCUGGGUGAA-3', the sequence is complemented with a partial sequence of VEGFA gene; and tracrRNA is the tracrRNA2 of the embodiment 1.

Table 3, in-vitro enzyme digestion reaction system of Cas9

| Reagent | Volume | Final concentration |
|---|---|---|
| Cas9 nuclease (1.2μM) | 2.5μl | 100nM |
| 10×Cas9Buffer | 3μl | 1× |
| crRNA(4μM) | 3μl | 400nM |
| tracrRNA(4μM) | 3μl | 400nM |
| DNA template | 2μl(192ng) | 20nM |
| $H_2O$ | 16.5μl | - |
| Total | 30μl | - |

3, Magnetic beads purification

1) 30 μl of an enzyme digestion product of the step 2 is transferred to an EP tube;
2) Twice the volume of magnetic beads is uniformly mixed with a sample in the EP tube by vortex oscillation, static-standing for 5-10 min;
3) The EP tube is placed on a magnetic force frame, after the magnetic beads are totally adhered to a side wall, supernatant is absorbed;
4) 200μl of 80% (percentage by volume) ethyl alcohol water solution is added to the EP tube, and the magnetic beads are eluted;
5) The supernatant is absorbed, static-standing for 3-5 min at room temperature, while the magnetic beads adhered to the wall are dried until a crack occurs, 20μl of water is added for dissolving;
6) After the water is added, the EP tube is taken out from the magnetic force frame, and static-standing for 3-5 min;
7) The EP tube is placed back to the magnetic force frame, and the supernatant is the recycled sample namely.

4, Agilent2200 nucleic acid automatic electrophoresis system detection

1-2μl of the sample and dye in equal volume are taken and detected on the machine.

5, Enzyme digestion result

(1) Calculating method

A DNA fragment distribution and concentration result is obtained through the Agilent2200 nucleic acid automatic electrophoresis system detection, and enzyme digestion efficiency is obtained through a calculating formula.

The calculating formula: Indel% (enzyme digestion efficiency) = $100\times(1-\sqrt{1-f(cut)})\%$ f(cut) = (b+c)/(a+b+c):a is the mole concentration (pmol/l) of an undigested fragment, b and c are the mole concentration (pmol/l) of digested fragments.

(2) Result analysis

FIG. 1 indicates that while a length of crRNA is 32nt(VEGFA-crRNA1), 34nt(VEGFA-crRNA2), 36nt(VEGFA-crRNA3) and 38nt(VEGFA-crRNA4), an in-vitro enzyme digestion system including the crRNA, tracrRNA and Cas9 may digest a DNA template of 485 bp. While the length of the crRNA is 34 nt, the Cas9 enzyme digestion effect is optimal (Indel% is 89.2%), namely while a length of a ncrRNA part is 14 nt (namely ncrRNA2), the Cas9 enzyme digestion efficiency is the highest.

II, Cas9 intracellular enzyme digestion detection

1, Cell transfection

(1) In culture conditions of 37 DEG C and 5% of $CO_2$, Cas9-293T cells are cultured in a culture box with a DMEM culture medium containing 10% of fetal calf serum. Herein, the Cas9-293T cells are stably imported with the Cas9 nuclease encoding gene through gene editing, and the Cas9 nuclease (sequence is a sequence shown in Genbank ID: AQM52323.1 on the NCBI) is expressed.
(2) In the day before transfection, the Cas9-293T cells for stably expressing the Cas9 nuclease are laid in a 24-pore plate.
(3) Cell density is adjusted to be about $4\times10^4$-$5\times10^4$ cells/mL, and lipo2000 (Lipofectamine2000) is used for transfecting the crRNA and the tracrRNA. Herein, the crRNA is the VEGFA-crRNA1, VEGFA-crRNA2, VEGFA-crRNA and VEGFA-crRNA4 of the step I, one type of the crRNA in each reaction system, and the tracrRNA is the tracrRNA2 of the embodiment 1. $H_2O$ for replacing the crRNA is used as a negative control (neg.). The method specifically includes the following steps:

A. 50μl of a opti-MEM culture medium is taken and placed in 1.5 mL of the EP tube, 1μl of the lipo2000 is added, gently mixing, and static-standing for 5 min at room temperature;
B. 50μl of the opti-MEM culture medium is taken and placed in 1.5 mL of the EP tube, 200 ng of the crRNA and 300 ng of the tracrRNA are added, adequately mixing;
C. solutions obtained in the steps A and B are mixed, and static-standing for 20 min at room temperature;

D. a part of the culture medium in the 24-pore culture plate in the step (1) is absorbed, the residual culture medium in pores is about 300 μl, and the solution obtained in the step C is added to the culture pores for uniformly mixing; and

E. after 3-6 h, the DMEM culture medium containing 10% of the fetal calf serum is replenished to the culture pores until 1 ml, and the cells are placed back to the culture box for culturing for 48 h, then the cell genomic DNA is extracted.

2, Cell genomic DNA extraction

(1) The DNA is extracted according to a specification of a TIANGEN blood/tissue/cell genomic DNA extracting kit.
(2) The DNA extracting effect is detected by agarose gel electrophoresis and DNA concentration and purity are tested.

3, Nested PCR amplification of the target DNA fragment

(1) Nested PCR amplification
The VEGFA target gene is amplified using Phusion enzyme. The primer used for the first PCR amplification is VEGFA-F1/VEGFA-R1, and the second PCR amplification is performed using the first PCR amplification product as the template, and using the primer VEGFA-F3/VEGFA-R3
(2) The product of the second PCR obtained in the step (1) is annealed, and an annealing product is directly used for the next step of enzyme digestion.

An annealing system is as follows, herein buffer2 is T7 Endonuclease I (NEB, article number: M0302L):

| | |
|---|---|
| PCR product | 5μl |
| buffer2 | 2μl |
| $H_2O$ | 12μl |

An annealing reaction is performed on a PCR instrument, and annealing conditions are as follows:

| | |
|---|---|
| 95 DEG C | 5 min |
| 95 DEG C to 85 DEG C | 2 DEG C/circulating |
| 85 DEG C to 25 DEG C | 0.1 DEG C/circulating |

4, T7EI enzyme digestion efficiency detection

The annealing product in the step 3 is enzymatic-digested by using NEB T7 endonuclease I (T7EI), after incubation at 37 DEG C for 40 min, 1 μl of protease K is added, and the incubation is performed for 5 min at 37 DEG C.

Enzyme digestion system (20 μl):

| | |
|---|---|
| Annealing product | 19 μl |
| T7EI | 1 μl |

5, Electrophoresis detection

An enzyme digestion sample is used for detection with the Agilent2200 nucleic acid automatic electrophoresis system.

6, Enzyme digestion result

(1) The calculating method is the same as the step I.
(2) Analysis

The result is shown in Fig. 2, it is indicated from the result that while a length of crRNA is 32nt (VEGFA-crRNA1), 34nt (VEGFA-crRNA2), 36nt (VEGFA-crRNA3) and 38nt (VEGFA-crRNA4), Cas9 may digest the target fragment of a target DNA of 485 bp. While the length of the crRNA is 34nt, the Cas9 enzyme digestion effect is optimal (Indel% is 29.4%), namely while a length of a ncrRNA part is 14 nt (namely ncrRNA2), the Cas9 enzyme digestion efficiency is the highest.

Embodiment 3, Effect of different tracrRNA lengths on DNA editing efficiency

I, In-vitro enzyme digestion detection

1, Method

According to the method in the step I of the embodiment 2, while crRNA is VEGFA-crRNA5, and tracrRNA is respectively tracrRNA1, tracrRNA2, tracrRNA3 and tracrRNA4 of the embodiment 1, the enzyme digestion efficiency of an in-vitro enzyme digestion system including the crRNA, tracrRNA and Cas9 is respectively detected, each reaction system includes one tracrRNA, and water for replacing tracrRNA is used as a negative control (neg.).

Herein, the sequence of the VEGFA-crRNA5 is UGACUGCCGUCUGCACACCC GUUUUAGAGCUAUG (SEQ ID NO.28).

2, Result

Through a DNA fragment distribution and concentration result (Fig. 3) obtained by Agilent2200 nucleic acid automatic electrophoresis system detection, a conclusion may be obtained: while a tracrRNA length is 64nt (tracrRNA1), 66nt (tracrRNA2), 68nt (tracrRNA3) and 70nt (tracrRNA4), Cas9 may digest a DNA template of 485 bp. It is known from an Indel % result that while the tracrRNA length is 66 nt (namely tracrRNA2), the Cas9 enzyme digestion effect is optimal, and Indel% is 90.7%.

II, Intracellular enzyme digestion detection

1, Method

According to the method in the step II of the embodiment 2, while the crRNA is the VEGFA-crRNA5 in the step I, and the tracrRNA is respectively the tracrRNA1, the tracrRNA2, the tracrRNA3 and the tracrRNA4 of the embodiment 1, the enzyme digestion efficiency of Cas 9 is respectively detected, each reaction system includes one tracrRNA, and the water for replacing the tracrRNA is used as the negative control (neg.).

2, Result

Through a DNA fragment distribution and concentration result (Fig. 4) obtained by the Agilent2200 nucleic acid automatic electrophoresis system detection, a conclusion may be obtained: while the tracrRNA length is 64nt (tracrRNA1), 66nt (tracrRNA2), 68nt (tracrRNA3) and 70nt (tracrRNA4), the Cas9 may digest a target fragment of 485 bp. It is known from the Indel% result that while the tracrRNA length is 66 nt (namely the tracrRNA2), the Cas9 enzyme digestion effect is optimal, and the Indel% is 29.8%.

Embodiment 4, Enzyme digestion detection of different modifications

I, In-vitro enzyme digestion detection

1, Method

The 293T cell genome is used as a template for amplifying a target gene VEGFA fragment, the specific method is the same as the embodiment 1.

According to the method of the step I of the embodiment 2, while crRNA respectively is VEGFA-crRNA2-1 and VEGFA-crRNA2-2, and tracrRNA is the tracrRNA2 of the embodiment 1, the enzyme digestion efficiency of an in-vitro enzyme digestion system including the crRNA, tracrRNA and Cas9 is respectively detected, each reaction system includes one crRNA. The VEGFA-crRNA2 of the embodiment 2 is used as a control, and $H_2O$ for replacing crRNA is used as a negative control (neg.).

Herein, the VEGFA-crRNA2-1 is a substance obtained by performing 2'-O-methyl modification on the 2nd site, the 30th site, the 31st site and the 33rd site of the crRNA5 of the embodiment 3, details are as follows:

UGmACUGCCGUCUGCACACCCGUUUUAGAGCmUmAUmG, m represents the 2'-O-methyl modification; and the VEGFA-crRNA2-2 is a substance obtained by performing cholesterol modification on crRNA2, details are as follows:
Chol-UGACUGCCGUCUGCACACCCGUUUUAGAGCUAUG, Chol represents the cholesterol modification.

2, Result

Through a DNA fragment distribution and concentration result obtained by Agilent2200 nucleic acid automatic electrophoresis system detection, it is known from Fig. 5 that after chemical modification (methyl modification VEGFA-crRNA2-1, cholesterol modification VEGFA-crRNA2-2) is performed on crRNA, Cas9 nuclease may digest a target fragment of 485 bp, and the enzyme digestion efficiency has no apparent change compared with the VEGFA-crRNA2.

II, Intracellular enzyme digestion detection

1, Method

According to the method of the step II of the embodiment 2, while the crRNA respectively is the VEGFA-crRNA2-1 and the VEGFA-crRNA2-2 of the step I, and the tracrRNA is the tracrRNA2 of the embodiment 1, the enzyme digestion efficiency of the Cas9 is respectively detected, and each reaction system includes one crRNA. The VEGFA-crRNA2 of the embodiment 2 is used as a control, and $H_2O$ for replacing the crRNA is used as a negative control (neg.).

2,Result

Through the DNA fragment distribution and concentration result obtained by Agilent2200 nucleic acid automatic electrophoresis system detection, it is known from Fig. 6 that after the chemical modification (methyl modification VEGFA-crRNA2-1, cholesterol modification VEGFA-crRNA2-2) is performed on the crRNA, a DNA template of 485 bp may be digested, and the enzyme digestion efficiency has no apparent change compared with the VEGFA-crRNA2.

Embodiment 5, Enzyme digestion detection of different cell lines

1, Method

According to the method in the step II of the embodiment 2, the Cas9-293T cell is respectively replaced by the Cas9-HeLa cell, the Cas9-THP-1 cell and the Cas9-HUVEC cell, the other steps are not changed, the effect of the different cell lines to the Cas9 enzyme digestion efficiency is detected, and each reaction system includes one type of the cell. Used crRNA is the VEGFA-crRNA2 of the embodiment 2, and tracrRNA is the tracrRNA2 of the embodiment 1, $H_2O$ for replacing tracrRNA is used as a negative control (neg.).

2, Result

Through a DNA fragment distribution and concentration result obtained by Agilent2200 nucleic acid automatic electrophoresis system detection, it is known from Fig. 7 that in the different types of the cell lines, after the crRNA and the tracrRNA are transfected, Cas9 nuclease may digest a target fragment, and a result of each negative control also shows that the enzyme digestion efficiency of the negative control is 0.

Embodiment 6, Enzyme digestion efficiency detection of different genes

Target gene: an organic cation/carnitine transporter 4 (Oct4) gene, an Empty spiracles homeobox 1 (EMX1) gene, a Beta-3 gene and a Beta-5 gene, herein both the Beta-3 and the Beta-5 are a β thalassemia gene, the Beta-3 gene contains a β thalassemia gene mutation site CD17 (HBB:c.52A>T), and the Beta-5 gene contains the β thalassemia gene mutation site CD17 (HBB:c.52A>T).

crRNA used for the Oct4 gene is Oct4-E-T1-crRNA, Oct4-E-T2-crRNA and Oct4-E-T3-crRNA, and tracrRNA is the tracrRNA2 of the embodiment 1. A structure of the crRNA is as shown in the formula I of the embodiment 1, the ncrRNA part of each crRNA is the same, and is the ncrRNA2 of the embodiment 1, Nx parts are different, and a sequence of each crRNA is as follows:

Oct4-E-T1-crRNA: GGUUAUUUCUAGAAGUUAGG GUUUUAGAGCUAUG(SEQ ID NO.13)
Oct4-E-T2-crRNA: CUUCUACAGACUAUUCCUUG GUUUUAGAGCUAUG(SEQ ID NO.14)
Oct4-E-T3-crRNA: GGAAAGGGGAGAUUGAUAAC GUUUUAGAGCUAUG(SEQ ID NO.15).

The crRNA used for the EMX1 gene is E1R-crRNA, E7F-crRNA, Ee3F-crRNA and E2F-crRNA, the tracrRNA is the tracrRNA2 of the embodiment 1. The structure of the crRNA is as shown in the formula I of the embodiment 1, the ncrRNA part of each crRNA is the same, and is the ncrRNA2 of the embodiment 1, the Nx parts are different, and the sequence of each crRNA is as follows:

E1R-crRNA: AAGGCCGUGCGGAUCCGCUU GUUUUAGAGCUAUG(SEQ ID NO.16)
E7F-crRNA: AGGUCCGACGUGUUGGAGUG GUUUUAGAGCUAUG(SEQ ID NO.17)
Ee3F-crRNA: CGAUGUCACCUCCAAUGACU GUUUUAGAGCUAUG(SEQ ID NO.18)
E2F-crRNA: UCUCGCCCUCGCAGCUGCUG GUUUUAGAGCUAUG(SEQ ID NO.19).

The crRNA used for the Beta-3 gene is Beta-3-T1-crRNA and Beta-3-T3-crRNA, the tracrRNA is the tracrRNA2 of the embodiment 1. The structure of the crRNA is as shown in the formula I of the embodiment 1, the ncrRNA part of each crRNA is the same, and is the ncrRNA2 of the embodiment 1, the Nx parts are different, and the sequence of each crRNA is as follows:

Beta-3-T1-crRNA : GUGUGGCAAAGGUGCCCUUG GUUUUAGAGCUAUG(SEQ ID NO.20).
Beta-3-T3-crRNA: ACCAAUAGAAACUGGGCAUG GUUUUAGAGCUAUG(SEQ ID NO.21).

The crRNA used for the Beta-5 gene is Beta-5-T3-crRNA, the tracrRNA is the tracrRNA2 of the embodiment 1. The structure of the crRNA is as shown in the formula I of the embodiment 1, and the sequence is as follows:
Beta-5-T3-crRNA: CGUAAAUACACUUGCAAAGG GUUUUAGAGCUAUG (SEQ ID NO.22).

I, In-vitro enzyme digestion detection

1, DNA template preparation

The 293T cell genome is used as a template to amplify fragments of the target gene of Oct4 gene, EMX1 gene, Beta-3 gene and Beta-5 gene, that is, a DNA template.

The primers used for the first amplification of the Oct4 gene DNA template are Oct4-outer-F1 and Oct4-outer-R1, and primers used for the second amplification are Oct4-inner-F1 and Oct4-inner-R1.

The EMX1 gene DNA template is EMX1-E2F, EMX1-E1R, EMX1-E7F and EMX1-Ee3F, the primer used for each template is as follows:
EMX1-E2F: the primers used for the first amplification of the DNA template are E1-1R/2F-WF/NF and E1-1R/2F-WR, and the primers used for the second amplification are E1-1R/2F-WF/NF and E1-1R/2F-NR.

EMX1-E1R: the primers used for the first amplification of the DNA template are E1-1R/2F-WF/NF and E1-1R/2F-WR, and the primers used for the second amplification are E1-1R/2F-WF/NF and E1-1R/2F-NR.

EMX1-E7F: the primers used for the first amplification of the DNA template are E1-7-F-WF and E1-7-F-WR, and the primers used for the second amplification are E1-7-F-NF and E1-7-F-NR.

EMX1-Ee3F: the primers used for the first amplification of the DNA template are E1-e3F-WF and E1-e3F-WR, and the primers used for the second amplification are E1-e3F-NF and E1-e3F-NR.

The primers used for the first amplification of the Beta-3 gene DNA template are Beta-outer-F1 and Beta-outer-R1, the primers used for the second amplification are Beta3-inner-F1 and Beta3-inner-R1.

The primers used for the first amplification of the Beta-5 gene DNA template are Beta-outer-F1 and Beta-outer-R1, the primers used for the second amplification are Beta5-inner-F1 and Beta5-inner-R1.

2, Cas9 in-vitro enzyme digestion

According to the methods 2-5 in the step I of the embodiment 2, an in-vitro enzyme digestion system containing crRNA, tracrRNA and Cas9 is used for performing enzyme digestion on each DNA template of the step 1, and each reaction system includes one crRNA. $H_2O$ for replacing crRNA is used as a negative control (neg).

2.1 Oct4 gene

The enzyme digestion efficiency of the in-vitro enzyme digestion system of Cas9 is detected in different concentration (200 nM, 400 nM and 800 nM) of Oct4-E-T1-crRNA, concentration of tracrRNA in each system is different along with different crRNA concentration, the concentration of the tracrRNA in the same reaction system is kept to be the same as the crRNA concentration, concentration of other substances is the same as Table 3.

It is displayed from the result that while the concentration of the Oct4-E-T1-crRNA is 200 nM, 400 nM and 800 nM, the Cas9 can digest the Oct4 gene DNA template (Fig. 8).

2.2 EMX1 gene

The enzyme digestion efficiency of the in-vitro enzyme digestion EMX1 gene DNA template of the Cas9 of different crRNAs (E1R-crRNA, E7F-crRNA, Ee3F-crRNA and E2F-crRNA) is detected, the crRNA corresponding to the EMX1 gene DNA template EMX1-E2F, EMX1-E1R, EMX1-E7F and EMX1-Ee3F respectively is E2F-crRNA, E1R-crRNA, E7F-crRNA and Ee3F-crRNA, and each system is the same as Table 3.

It is displayed from the result that the different crRNAs can digest the EMX1 gene DNA template (Fig. 9).

2.3 Beta-3 gene and Beta-5 gene

The enzyme digestion efficiency of the in-vitro enzyme digestion Beta-3 gene DNA template of the Cas9 in the presence of Beta-3-T1-crRNA and Beta-3-T3-crRNA is detected, and the enzyme digestion efficiency of the in-vitro enzyme digestion Beta-5 gene DNA template of the Cas9 in the presence of Beta-5-T3-crRNA is detected.

It is displayed from the result that in the presence of Beta-3-T1-crRNA and Beta-3-T3-crRNA, the Cas9 can digest the Beta-3 gene DNA template, and in the presence of Beta-5-T3-crRNA, the Cas9 may digest Beta-5 gene DNA template (Fig. 10).

II, Intracellular enzyme digestion detection

According to the method in the step II of the embodiment 2, the enzyme digestion efficiency of Cas9 nuclease to the Oct4 gene, the EMX1 gene, the Beta-3 gene and the Beta-5 gene is detected, and each reaction system includes one crRNA. $H_2O$ for replacing crRNA is used as a negative control (neg.).

The crRNA used for the Oct4 gene is Oct4-E-T1-crRNA, Oct4-E-T2-crRNA and Oct4-E-T3-crRNA, and the method is the same as the step II of the embodiment 2. In a nested PCR amplification of the target DNA fragment, the primers used for the Oct4 gene are as follows: the primers used for the first amplification are Oct4-outer-F1 and Oct4-outer-R1, and the primers used for the second amplification are Oct4-inner-F1 and Oct4-inner-R1. It is displayed from the result that in the presence of different crRNAs, Cas9 nuclease can digest the Oct4 gene (Fig. 11).

The crRNA used for the EMX1 gene is Ee3F-crRNA, a dosage of the Ee3F-crRNA respectively is 50 ng, 100 ng, 200 ng and 400 ng, concentration of tracrRNA in the same reaction system is kept to be the same as crRNA concentration, a dosage of other substances and a method are the same as the step II of the embodiment 2. In the target DNA fragment of the nested PCR amplification, the primers used for the EMX1 gene are as follows: the primers used for the first amplification are E1-e3F-WF and E1-e3F-WR, and the primers used for the second amplification are E1-e3F-NF and E1-e3F-NR. It is displayed from the result that under the dosages of different crRNAs, the Cas9 nuclease can digest the EMX1 gene (Fig. 12).

The crRNA used for the Beta-3 gene is Beta-3-T1-crRNA and Beta-3-T3-crRNA, the crRNA used for the Beta-5 gene is Beta-5-T3-crRNA, and the methods are the same as the step II of the embodiment 2. In the target DNA fragment of the nested PCR amplification, the primers used for the Beta-3 gene are as follows: the primers used for the first amplification are Beta-outer-F1 and Beta-outer-R1, and the primers used for the second amplification are Beta3-inner-F1 and Beta3-inner-R1; and the primers used for the Beta-5 gene are as follows: the primers used for the first amplification are Beta-outer-F1 and Beta-outer-R1, and the primers used for the second amplification are Beta5-inner-F1 and Beta5-inner-R1. It is displayed from the result that in the presence of the Beta-3-T1-crRNA and Beta-3-T3-crRNA, the Cas9 nuclease can digest the Beta-3 gene, and in the presence of the Beta-5-T3-crRNA, the Cas9 nuclease can digest the Beta-5 gene (Fig. 13).

Embodiment 7, Mouse genome VEGFA gene enzyme digestion detection

I, Intracellular enzyme digestion detection

According to the method in the step II of the embodiment 2, the Cas9-293T cell is replaced by the C2C12 mouse myoblast containing Cas9, other steps are not changed, the effect of Cas9 on enzyme digestion efficiency of VEGFA gene in C2C12 mouse myoblast containing Cas9 is detected, used crRNA is a modified VEGFA-crRNA6, namely VEGFA-crRNA6-1, and tracrRNA is the tracrRNA2 of the embodiment 1. $H_2O$ for replacing crRNA is used as a negative control (neg.).

VEGFA-crRNA6: AAGAGGAGAGGGGGCCGCAG GUUUUAGAGCUAUG(SEQ ID NO.29).

The VEGFA-crRNA6-1 is a substance obtained by performing 2'-O-methyl modification on the 2nd site, the 30th site, the 31st site and the 33rd site of the VEGFA-crRNA6, and details are as follows:

AAmGAGGAGAGGGGGCCGCAG GUUUUAGAGCmUmAUmG, m represents the 2'-O-methyl modification.

A preparation method for the C2C12 mouse myoblast containing the Cas9 is as follows:

1, Transfecting plasmid PX260 by Lipo2000

(1) 50μl of an opti-MEM culture medium is taken and placed in 1.5 ml of an EP tube, 1μl of the lipo2000 is added, after uniformly mixing, static-standing for 5 min at room temperature;
(2) 50μl of the opti-MEM culture medium is taken and placed in 1.5 ml of the EP tube, 1μg of the plasmid PX260 is added, and uniformly mixing;
(3) solutions obtained in steps (1) and (2) are mixed, static-standing for 20 min at room temperature; and
(4) a part of a culture medium in a 24-pore culture plate for culturing the C2C12 mouse myoblast is absorbed, the residual culture medium in pores is about 300-500μl, the sample of the step (3) is added to the culture pores, after 3-6 h, a DMEM culture medium containing 10% of fetal calf serum is replenished to the culture pores until 1 ml, and the cells are placed back to a culture box for culturing.

2, After transfecting the plasmid for 24 h, crRNA and tracrRNA are transfected with Lipo2000. The transfection method is the same as (3) of 1 in the step I of the embodiment 2.

II, C57bl/6 mouse in-vivo enzyme digestion detection

1, C57bl/6 mouse in-vivo transfection:

(1) A C57bl/6 mouse (Beijing Charles River Labs) is injected with pentobarbital sodium for anesthesia: 30 mg/kg of intravenous or intraperitoneal injection.
(2) Peparation mixing

5μg of a Cas9 plasmid (namely PX260), 4μM of crRNA (20μl) and 4μM of tracrRNA (20μl) are dissolved in 200 μl of normal saline, and oscillating and mixing uniformly. 6 μl of a transfection reagent (namely TurboFect in vivo Transfection Reagent) is added and mixed adequately. The above mixed solution is placed for 15-20 minutes at room temperature, and used as a transfection preparation for intramuscular injection. Used crRNA is a modified VEGFA-crRNA6, namely VEGFA-crRNA6-1, and tracrRNA is the tracrRNA2 of the embodiment 1.

2.2 Mouse intramuscular injection

200μl of the transfection preparation in the step 2.1 is injected to mouse biceps.

2.3 Mouse muscular tissue genomic DNA extraction

In the seventh day after transfecting, a blood/tissue/cell genome extracting kit is used for extracting biceps genome DNA.

2.4 Nested PCR amplification of the target DNA fragment

A method is the same as 3 in the step II of the embodiment 2.Primers used are VEGFA-outer-F1 (mouse), VEGFA-outer-R1 (mouse), VEGFA-inner-F1 (mouse) and VEGFA-inner-R1 (mouse).

2.5 T7EI enzyme digestion efficiency detection

NEB T7 endonuclease I(T7EI) is used for performing enzyme digestion, a specific method is the same as 4 in the step II of the embodiment 2.

2.6 Electrophoresis detection

An enzyme digestion sample is used for Agilent2200 nucleic acid automatic electrophoresis system detection.

3, Result analysis

A calculating method is the same as the embodiment 2.

Through a DNA fragment distribution and concentration result obtained by the Agilent2200 nucleic acid automatic electrophoresis system detection, it is known that in the mouse, the crRNA, tracrRNA and Cas9 plasmid are imported into a mouse cell or a mouse body, a target fragment of 526 bp of a target DNA can be digested into two DNA chains (Fig. 14).

Embodiment 8, Promoter and exon enzyme digestion detection

The enzyme digestion efficiency of TP53 (tumor protein p53) gene and an exons is detected, the used crRNA respectively is TP53-P-T1, TP53-P-T2, TP53-E-T1, TP53-E-T2 and TP53-E-T3, each reaction system includes one crRNA, the used tracrRNA is the tracrRNA2 of the embodiment 1. $H_2O$ for replacing crRNA is used as a negative control (neg.). A sequence of the used crRNA is as follows:

TP53-P-T1 : CAAUUCUGCCCUCACAGCUC GUUUUAGAGCUAUG(SEQ ID NO.23).
TP53-P-T2 : CCCCAAAAUGUUAGUAUCUA GUUUUAGAGCUAUG(SEQ ID NO.24).
TP53-E-T1 : CCCUCCCAUGUGCUCAAGAC GUUUUAGAGCUAUG(SEQ ID NO.25).
TP53-E-T2: UGGGAGCGUGCUUUCCACGA GUUUUAGAGCUAUG(SEQ ID NO.26)
TP53-E-T3: CCAGUCUUGAGCACAUGGGA GUUUUAGAGCUAUG(SEQ ID NO.27).

(P represents the promoter, and E represents the exon).

I, In-vitro enzyme digestion detection

1, DNA template preparation

The 293T cell genome is used as a template for amplifying a target gene TP53 fragment, namely a DNA template.

Primers used for the first amplification are TP53-outer-FP and TP53-outer-RP, and the primers used for the second amplification are TP53-inner-FP and TP53-inner-RP.

2. Cas9 in-vitro enzyme digestion

According to the methods of 2-5 in the step I of the embodiment 2, an in-vitro enzyme digestion system containing crRNA, tracrRNA and Cas9 is used for performing enzyme digestion on the DNA template in the step 1.

II, Intracellular enzyme digestion detection

According to the method in the step II of the embodiment 2, the enzyme digestion efficiency of Cas9 nuclease to a TP53 fragment is detected. $H_2O$ for replacing crRNA is used as a negative control (neg.).

Through a DNA fragment distribution and concentration result obtained by Agilent2200 nucleic acid automatic electrophoresis system detection, it is known from Fig. 15 to FIG. 16 that crRNA, tracrRNA and Cas9 nuclease systems can digest promoter and exon areas of the TP53 fragment.

Embodiment 9, Enzyme digestion detection of multiple crRNAs

I, In-vitro enzyme digestion detection

1, Cas9 in-vitro enzyme digestion

Cas9 nuclease, 10×Cas9 buffer, crRNA, tracrRNA and $H_2O$ are uniformly mixed, and incubated at 37 DEG C for 15 min, after that, the DNA template is added and mixed uniformly, then the incubation is performed for 70 min at 37 DEG C. The $H_2O$ for replacing crRNA is used as a negative control (neg.). The DNA template is the Oct4 gene DNA template of the embodiment 6, crRNAs used are Oct4-E-T1-crRNA, Oct4-E-T2-crRNA and Oct4-E-T3-crRNA, and tracrRNA is the tracrRNA2 of the embodiment 1. A Cas9 in-vitro enzyme digestion system is as follows, the system contains three types of the crRNAs, namely Oct4-E-T1-crRNA, Oct4-E-T2-crRNA and Oct4-E-T3-crRNA:

| Reagent | Volume | Final concentration |
|---|---|---|
| Cas9 nuclease (1.2μM) | 2.5μl | 100nM |
| 10×Cas9 Buffer | 3μl | 1× |
| crRNA(all are 4μM) | Oct4-E-T1-crRNA 1 μl<br>Oct4-E-T2-crRNA 1 μl<br>Oct4-E-T3-crRNA 1 μl | 133.3nM<br>133.3nM<br>133.3nM |
| tracrRNA(4μM) | 3μl | 400nM |
| DNA template | 2μl 192ng | 20nM |
| $H_2O$ | 16.9μl | - |
| total | 30μl | - |

The Oct4-E-T2-crRNA is used alone as the crRNA for performing the experiment, as a comparison, a concentration of the Oct4-E-T2-crRNA is 400 nM.

2, Magnetic beads purification

Same as 3 in the step I of the embodiment 2.

3, Agilent2200 nucleic acid automatic electrophoresis system detection

Same as 4 in the step I of the embodiment 2.

II, Intracellular enzyme digestion detection

According to the method in the step II of the embodiment 2, the Oct4-E-T1-crRNA, the Oct4-E-T2-crRNA, the Oct4-E-T3-crRNA and the tracrRNA2 of the embodiment 1 are used for transfecting together, a dosage of the crRNA6 is 66.7 ng, and a dosage of the tracrRNA2 is 300 ng. The Oct4-E-T2-crRNA is used alone as the crRNA for performing the experiment, as a comparison, a dosage of the Oct4-E-T2-crRNA is 200 ng.

Through a DNA fragment distribution and concentration result obtained by the Agilent2200 nucleic acid automatic electrophoresis system detection, it is known from Figs. 17-18 that in the case of the consistent crRNA dosage, the enzyme digestion efficiency of multiple crRNAs in allusion to the Oct 4 gene is higher than that of the single crRNA, in-vitro and intracellular enzyme digestion efficiency is respectively improved by 1 time and twice or more.

Embodiment 10, Cell enzyme digestion detection of different Cas9 donors

I, Cells for stably expressing Cas9

The Cas9 donor is a 293T cell for stably expressing Cas9, namely Cas9-293T, a specific method is the same as the step I of the embodiment 2. Used crRNA and tracRNA respectively are the crRNA2 of the embodiment 2 and the tracrRNA2 of the embodiment 1.

2, Cas9 plasmid

The Cas9 donor is a Cas9 plasmid (namely PX260).

2.1 Transfecting plasmid PX260 by Lipo2000

(1) 50μl of an opti-MEM culture medium is taken and placed in 1.5 ml of an EP tube, 1μl of the lipo2000 is added, after uniformly mixing, static-standing for 5 min at room temperature;
(2) 50μl of the opti-MEM culture medium is taken and placed in 1.5 ml of the EP tube, 1μg of the plasmid PX260 is added, and uniformly mixing;
(3) solutions obtained in steps (1) and (2) are mixed, static-standing for 20 min at room temperature; and
(4) a part of a culture medium in a 24-pore culture plate for culturing the 293T cell is absorbed, the residual culture medium in pores is about 300-500μl, a sample of the step (3) is added to the culture pores, after 3-6 h, a DMEM culture medium containing 10% of fetal calf serum is replenished to the culture pores until 1 ml, and the cells are placed back to a culture box for culturing.

2.2, After transfecting the plasmid for 24 h, crRNA and tracrRNA are transfected by the Lipo2000. A transfection method is the same as (3) of 1 in the step I of the embodiment 2. The used crRNA and tracRNA respectively are the crRNA2 of the embodiment 2 and the tracrRNA2 of the embodiment 1.

According to the method of 2-6 in the step II of the embodiments, through a DNA fragment distribution and concentration result obtained by Agilent2200 nucleic acid automatic electrophoresis system detection, it is known from Fig. 19 that the normal 293T cell may enzymatic-digest a target DNA fragment too after the plasmid is transfected for expressing Cas9, and crRNA and tracrRNA are transfected, the efficiency is lower than that of a Cas9 stable transfecting cell (Cas9-293T).

Embodiment 11, In-vitro enzyme digestion detection of different Cas9 concentration

1, Method

According to the method in the step I of the embodiment 2, the enzyme digestion efficiency of an in-vitro enzyme digestion system containing crRNA, tracrRNA and different concentration of Cas9 is respectively detected. The concentration of the Cas9 respectively is 20 nM, 100 nM, 200 nM and 400 nM, and content of other components is the same as the step I of the embodiment 2.
Used crRNA and tracRNA respectively are the VEGFA-crRNA2 of the embodiment 2 and the tracrRNA2 of the embodiment 1.

2, Result

Through a DNA fragment distribution and concentration result (Fig. 20) obtained by Agilent2200 nucleic acid automatic electrophoresis system detection, a conclusion may be obtained: while protein concentration is 20-400 nM, the enzyme digestion efficiency is more than 50%; while the protein concentration is 100-400 nM, the enzyme digestion efficiency is more than 80%; and while the protein concentration is 100-200 nM, the enzyme digestion efficiency is more than 85%.

## Claims

**1.** A system for DNA editing, comprising A or B:
A, the following N1) and N2):

N1) is a RNA named as RNA-2 or a biological material related to the RNA-2;
N2) is a RNA named as RNA-1 or a biological material related to the RNA-1;

The RNA-2 is the RNA as shown in a formula I:

Nx-ncrRNA

The formula I:
wherein, the Nx is a spacer in the CRISPR/Cas system;
The ncrRNA is any one of the following a1) to a4):

a1) a RNA as shown in SEQ ID NO.1 of the sequence listing;
a2) a RNA acquired by adding one or more nucleotides to 5'-terminal and/or 3'-terminal of the a1);
a3) a RNA having more than 85% of identity with the RNA defined in a1) or a2); and
a4) a modifier of the RNA as shown in SEQ ID NO.1 of the sequence listing.

The biological material related to the RNA-2 is any one of the following A1) to A5:

A1) a DNA molecule for encoding the RNA-2;
A2) an expression cassette containing the DNA molecule of A1);
A3) a recombinant vector containing the DNA molecule of A1), or a recombinant vector containing the expression cassette of A2);
A4) a recombinant microorganism containing the DNA molecule of A1), or a recombinant microorganism containing the expression cassette of A2), or a recombinant microorganism containing the recombinant vector of A3); and
A5) a cell line containing the DNA molecule of A1), or a cell line containing the expression cassette of A2).

The RNA-1 is any one of the following b1) to b4):

b1) a RNA as shown in SEQ ID NO.5 of the sequence listing;
b2) a RNA acquired by adding one or more nucleotides to 5'-terminal and/or 3'-terminal of the b1);
b3) a RNA having more than 85% of identity with the RNA defined in b1) or b2); and
b4) a modifier of the RNA as shown in SEQ ID NO.5 of the sequence listing.

The biological material related to the RNA-1 is any one of the following B1) to B5:

B1) a DNA molecule for encoding the RNA-1;
B2) an expression cassette containing the DNA molecule of B1);
B3) a recombinant vector containing the DNA molecule of B1), or a recombinant vector containing the expression cassette of B2);
B4) a recombinant microorganism containing the DNA molecule of B1), or a recombinant microorganism containing the expression cassette of B2), or a recombinant microorganism containing the recombinant vector of B3); and
B5) a cell line containing the DNA molecule of 1), or a cell line containing the expression cassette of B2).

The ncrRNA is partially complemented to the RNA-1.
B, the RNA-2.

**2.** The system as claimed in claim 1, wherein the RNA of a2) is any one of the following a21) to a23):

a21) a 12-14nt RNA;
a22) a 12-16nt RNA;
a23) a 12-18nt RNA;

and/or, the RNA of b2) is any one of the following b21) to b24):

b21) a 64-66nt RNA;
b22) a 64-68nt RNA;
b23) a 64-70nt RNA;
b24) a 64-86nt RNA;

and/or, the modifier of the RNA is a substance obtained by modification of ribonucleotide on a ribose, a phosphate skeleton and/or a basic group; the modifier is a substance obtained by modification at least one nucleotide in the RNA on the ribose, the phosphate skeleton and/or the basic group;
and/or,
the DNA is any one of the following M1)-M5):

M1) an eukaryote DNA;
M2) an animal DNA;
M3) a mammalian DNA;
M4) a human DNA; and
M5) a mouse DNA.

**3.** The system as claimed in claim 1 or 2, wherein:

the RNA of a21) is the RNA as shown in SEQ ID NO.2 of the sequence listing;
the RNA of a22) is the RNA as shown in SEQ ID NO.3 of the sequence listing;
the RNA of a23) is the RNA as shown in SEQ ID NO.4 of the sequence listing;
and/or, the RNA of b21) is the RNA as shown in SEQ ID NO.6 of the sequence listing;
the RNA of b22) is the RNA as shown in SEQ ID NO.7 of the sequence listing;
the RNA of b23) is the RNA as shown in SEQ ID NO.8 of the sequence listing;
and/or,
the modification is 2'-O-methyl modification, 2'-deoxidation modification, 2'-fluorination modification or cholesterol modification;
and/or,
the DNA is a VEGFA gene, an EMX1 gene, an Oct4 gene, a Beta-3 gene, a Beta5 gene, a TP53 gene or a promoter of the TP53 gene.

**4.** The system as claimed in claim 1-3, wherein:

while the DNA is the VEGFA gene, the RNA-2 is the RNA as shown in at least one sequence of SEQ ID NO.9-12, SEQ ID NO.28 and SEQ ID NO.29;
while the DNA is the Oct4 gene, the RNA-2 is the RNA as shown in at least one sequence of SEQ ID NO.13-15;
while the DNA is the EMX1 gene, the RNA-2 is the RNA as shown in at least one sequence of SEQ ID NO.16-19;
while the DNA is the β thalassemia gene, the RNA-2 is the RNA as shown in SEQ ID NO. 20-22;
while the DNA is the TP53 gene, the RNA-2 is the RNA as shown in SEQ ID NO.23 and/or 24; and
while the DNA is the promoter of the TP53 gene, the RNA-2 is the RNA as shown in at least one sequence of SEQ ID NO.25-27.

**5.** The system as claimed in claims 1-4, wherein the system further comprises Cas9 nuclease or a biological material related to the Cas9 nuclease; and
the biological material related to the Cas9 nuclease is any one of the following C1) to C7):

C1) a nucleic acid molecule for encoding the Cas9 nuclease;
C2) an expression cassette containing the nucleic acid molecule of C1);
C3) a recombinant vector containing the nucleic acid molecule of C1), or a recombinant vector containing the expression cassette of C2);
C4) a recombinant microorganism containing the nucleic acid molecule of C1), or a recombinant microorganism containing the expression cassette of C2), or a recombinant microorganism containing the recombinant vector of C3); and
C5) a cell line containing the nucleic acid molecule of C1), or a cell line containing the expression cassette of C2).

**6.** The system as claimed in claim 5, wherein the Cas9 nuclease is derived from R1) or R2):

R1) bacteria; and
R2) streptococcus, staphylococcus, rothia, neisseria, corynebacterium or lactobacillus.

**7.** The following O1) or O2):

O1) the RNA-2 or a biological material related to the RNA-2 as claimed in any one of claims 1-4; and
O2) the RNA-1 or a biological material related to the RNA-1 as claimed in any one of claims 1-3.

**8.** Any one of the following applications:

X1. Use of the ncrRNA as claimed in any one of claims 1-4 in preparing the crRNA;
X2. Use of the ncrRNA as claimed in any one of claims 1-4 in DNA editing;
X3. Use of the ncrRNA as claimed in any one of claims 1-4 in preparing a DNA editing product;
X4. Use of the RNA-1 as claimed in any one of claims 1-3 in serving as tracrRNA;
X5. Use of the RNA-1 or the biological material related to the RNA-1 as claimed in any one of claims 1-3 in DNA editing;
X6. Use of the RNA-1 or the biological material related to the RNA-1 as claimed in any one of claims 1-3 in preparing a DNA editing product;
X7. Use of the RNA-2 as claimed in any one of claims 1-4 in serving as crRNA;
X8. Use of the RNA-2 or the biological material related to the RNA-2 as claimed in any one of claims 1-4 in DNA editing;
X9. Use of the RNA-2 or the biological material related to the RNA-2 as claimed in any one of claims 1-4 in preparing a DNA editing product;
X10. Use of the system as claimed in any one of claims 1-6 in DNA editing;
X11. Use of the system as claimed in any one of claims 1-6 in interfering gene expression in-vivo or in-vitro; and
X12. Use of the system as claimed in any one of claims 1-6 in establishing animal, plant or cell models of which the DNA is edited.

**9.** A method for editing DNA, comprising: processing the DNA to realize the DNA editing with the system as claimed in any one of claims 1-6.

**10.** A preparation method for the system as claimed in any one of claims 1-6, comprising independently packaging each substance in the system.

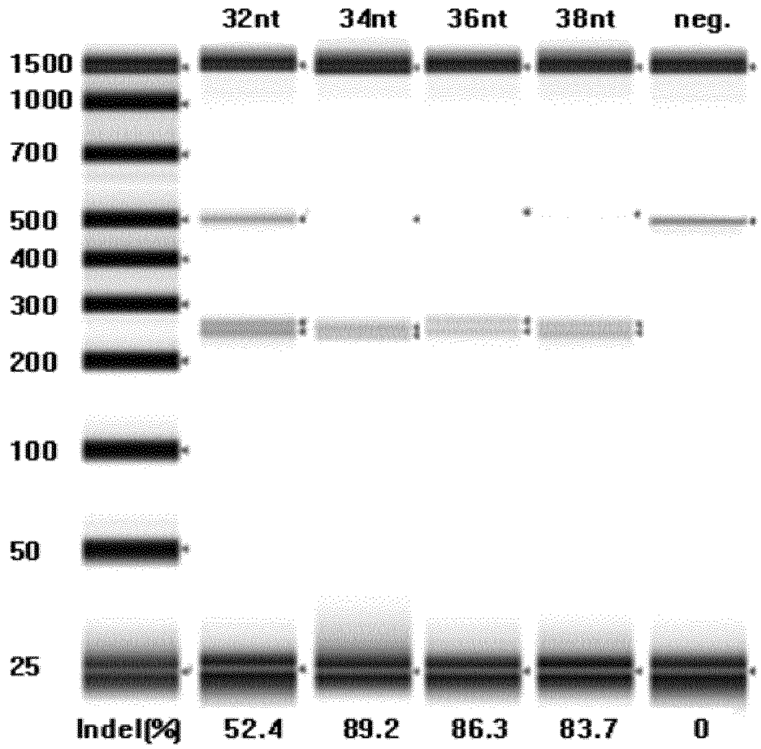
32nt
34nt
36nt
38nt
neg.
1500
1000
700
500
400
300
200
100
50
25
Indel(%)
52.4
89.2
86.3
83.7
0

Fig. 1

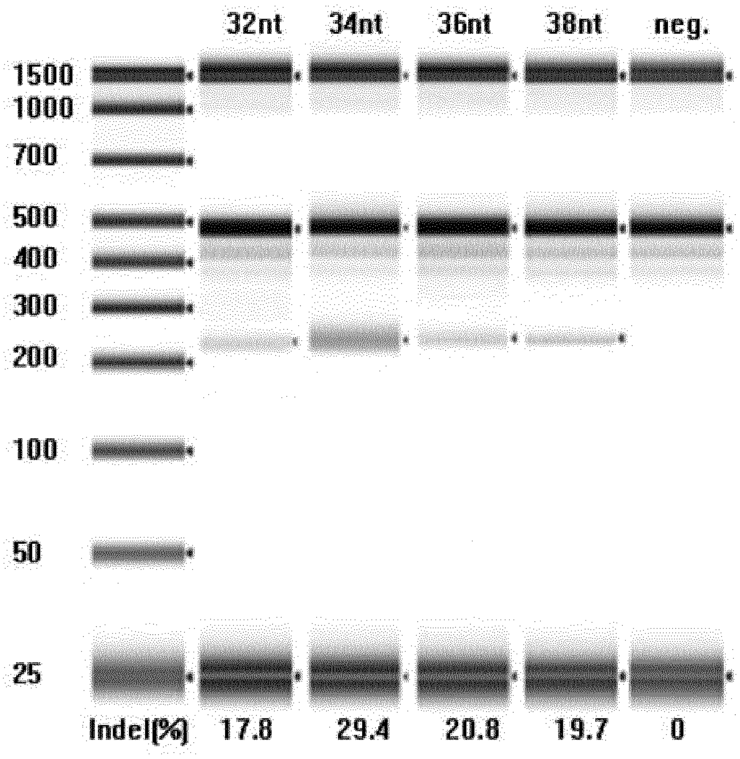
32nt
34nt
36nt
38nt
neg.
1500
1000
700
500
400
300
200
100
50
25
Indel(%)
17.8
29.4
20.8
19.7
0

Fig. 2

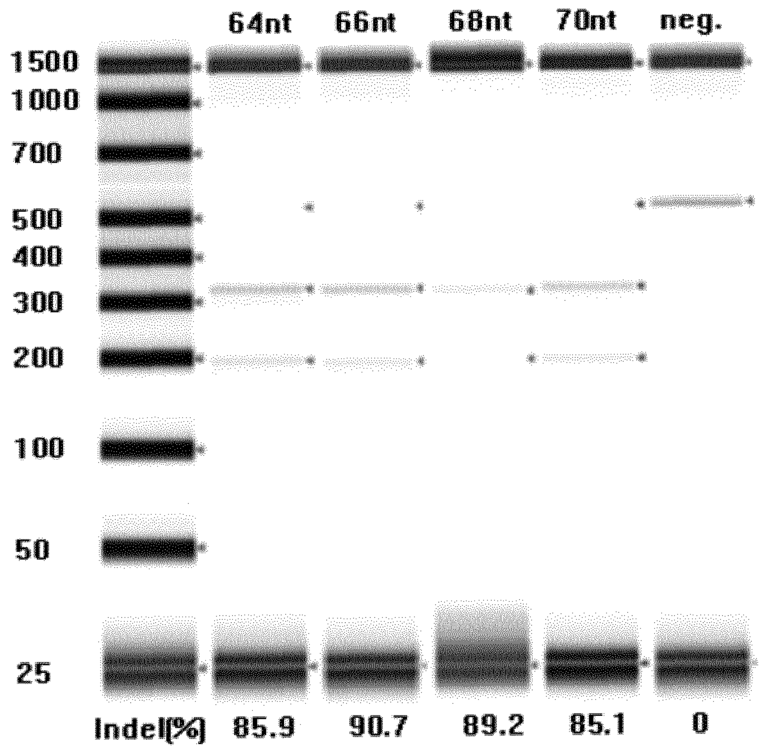
64nt
66nt
68nt
70nt
neg.
1500
1000
700
500
400
300
200
100
50
25
Indel(%)
85.9
90.7
89.2
85.1
0

Fig. 3

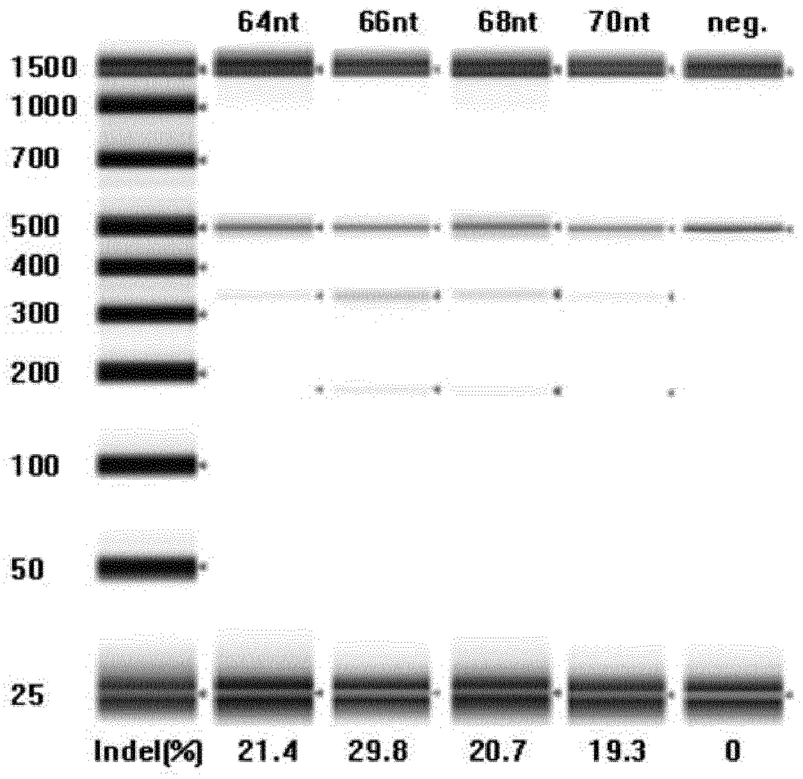
64nt
66nt
68nt
70nt
neg.
1500
1000
700
500
400
300
200
100
50
25
Indel(%)
21.4
29.8
20.7
19.3
0

Fig. 4

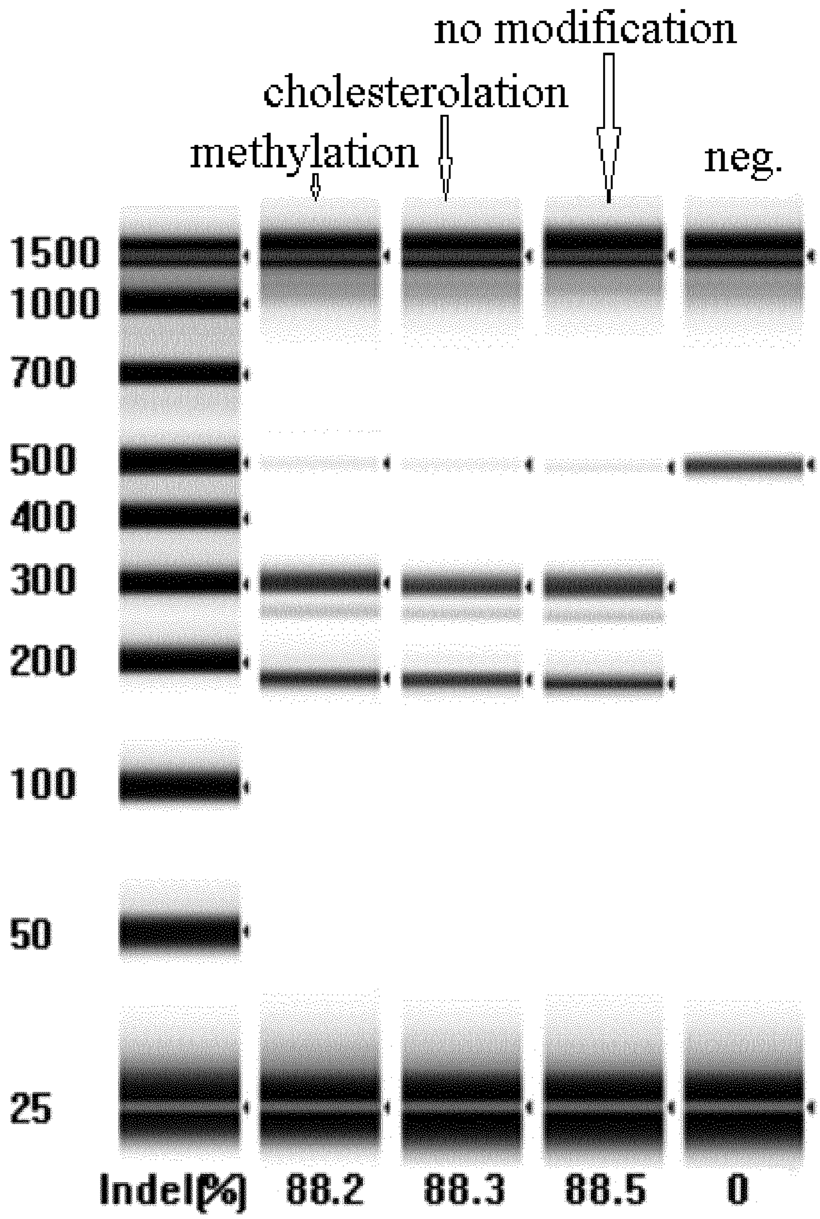
no modification
cholesterolation
methylation
neg.
1500
1000
700
500
400
300
200
100
50
25
Indel(%)
88.2
88.3
88.5
0

Fig. 5

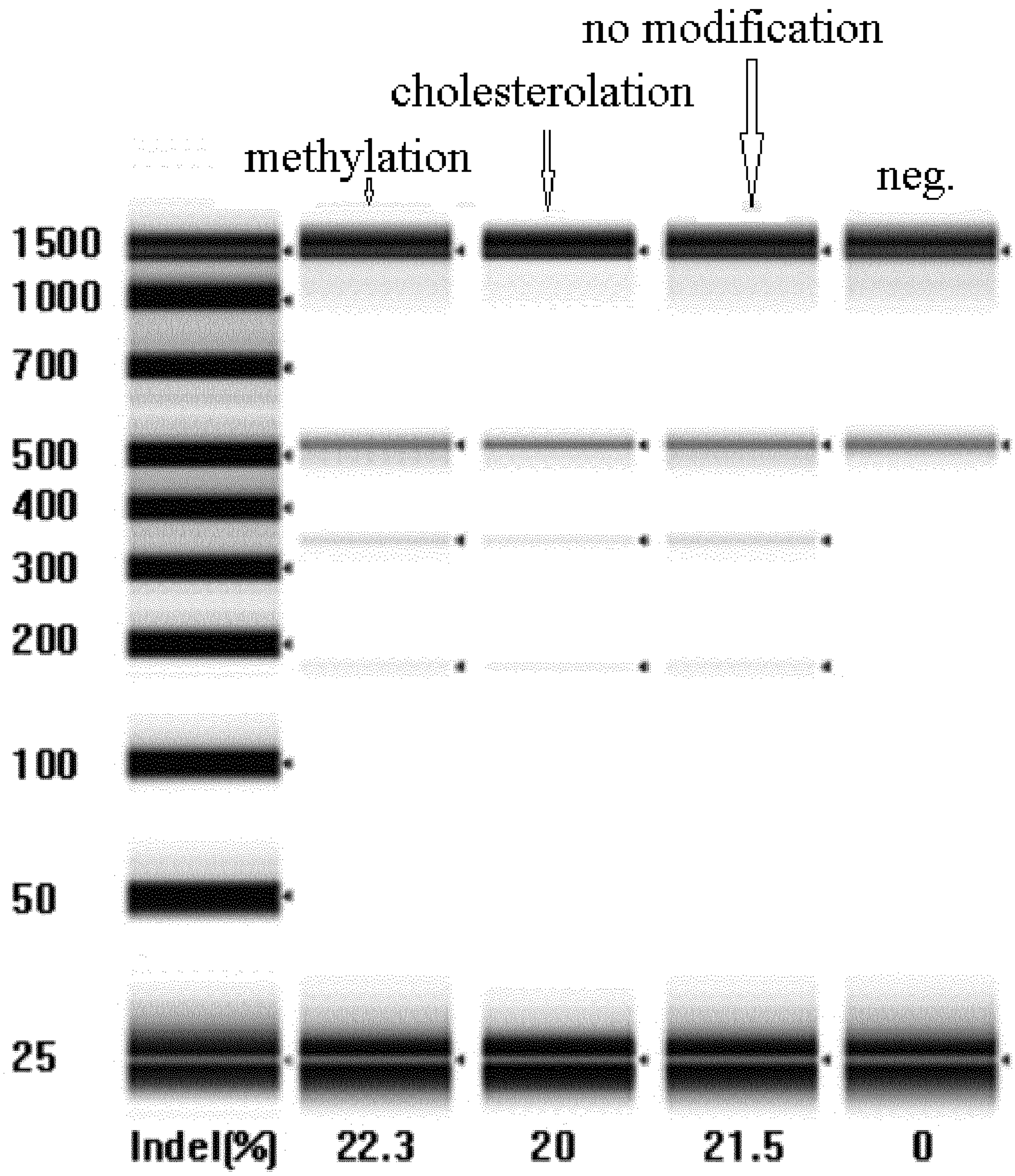
no modification
cholesterolation
methylation
neg.
1500
1000
700
500
400
300
200
100
50
25
Indel(%)
22.3
20
21.5
0

Fig. 6

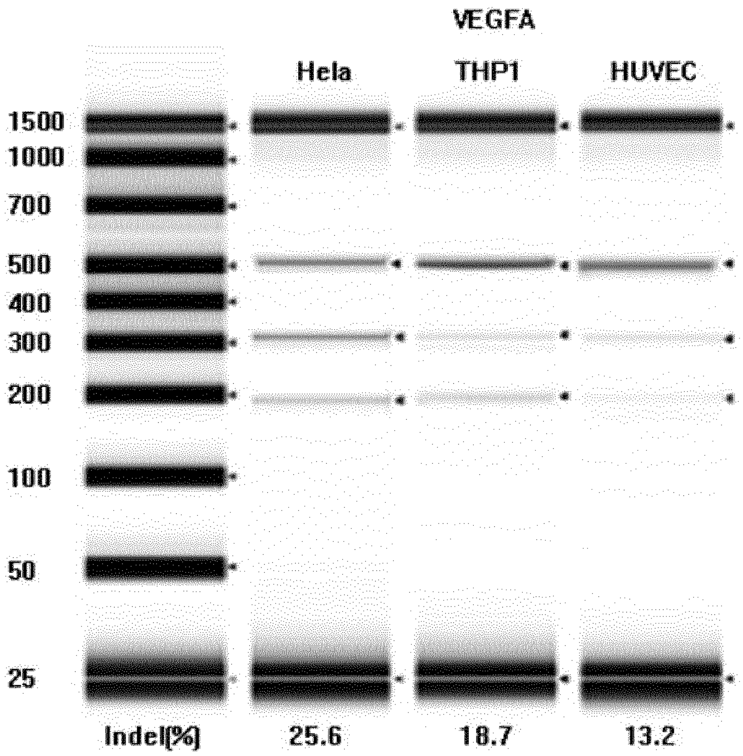
VEGFA
Hela
THP1
HUVEC
1500
1000
700
500
400
300
200
100
50
25
Indel(%)
25.6
18.7
13.2

Fig. 7

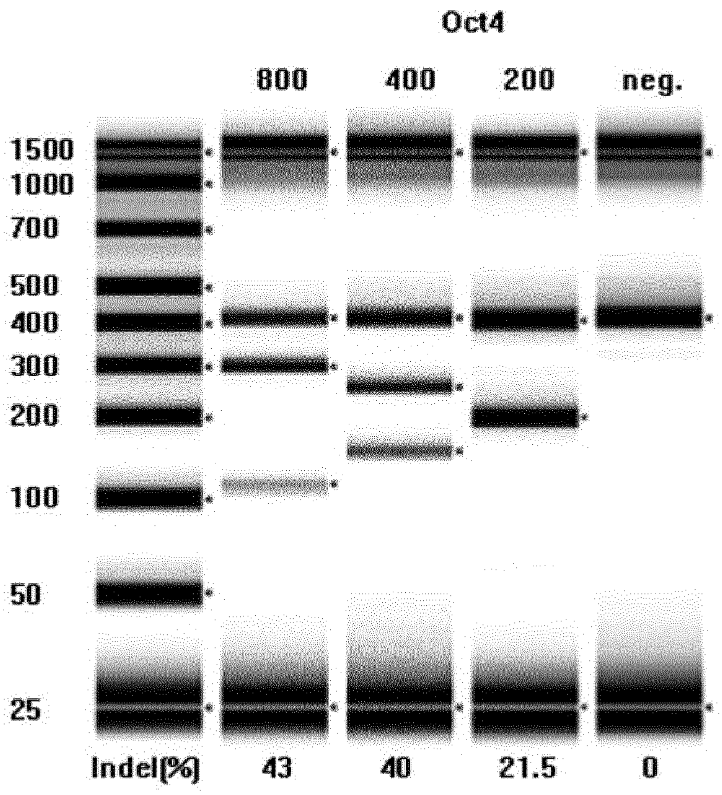
Oct4
800
400
200
neg.
1500
1000
700
500
400
300
200
100
50
25
Indel(%)
43
40
21.5
0

Fig. 8

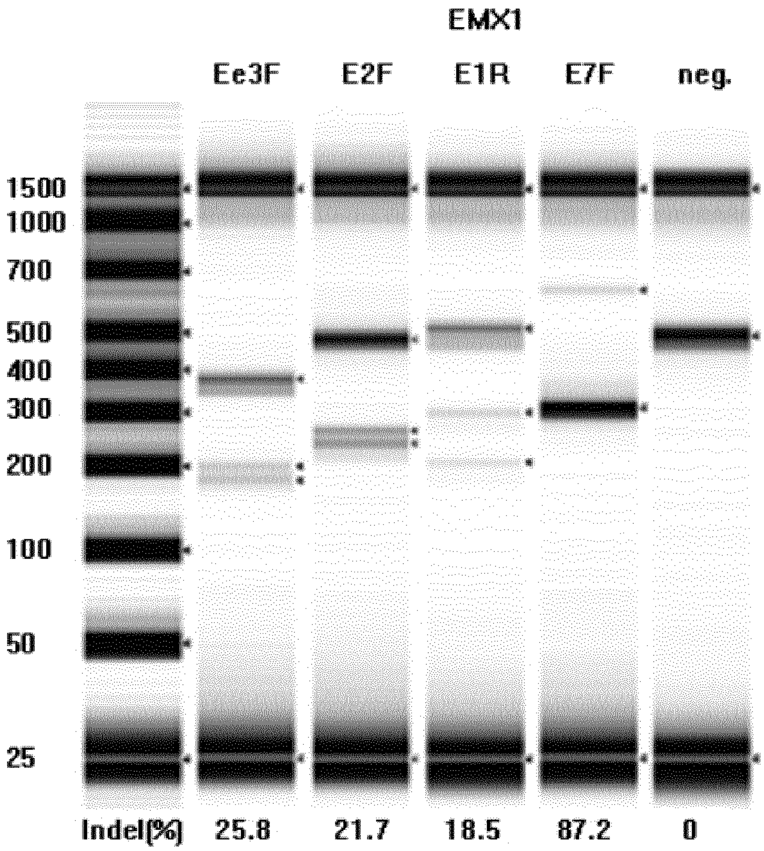
EMX1
Ee3F
E2F
E1R
E7F
neg.
1500
1000
700
500
400
300
200
100
50
25
Indel(%)
25.8
21.7
18.5
87.2
0

Fig. 9

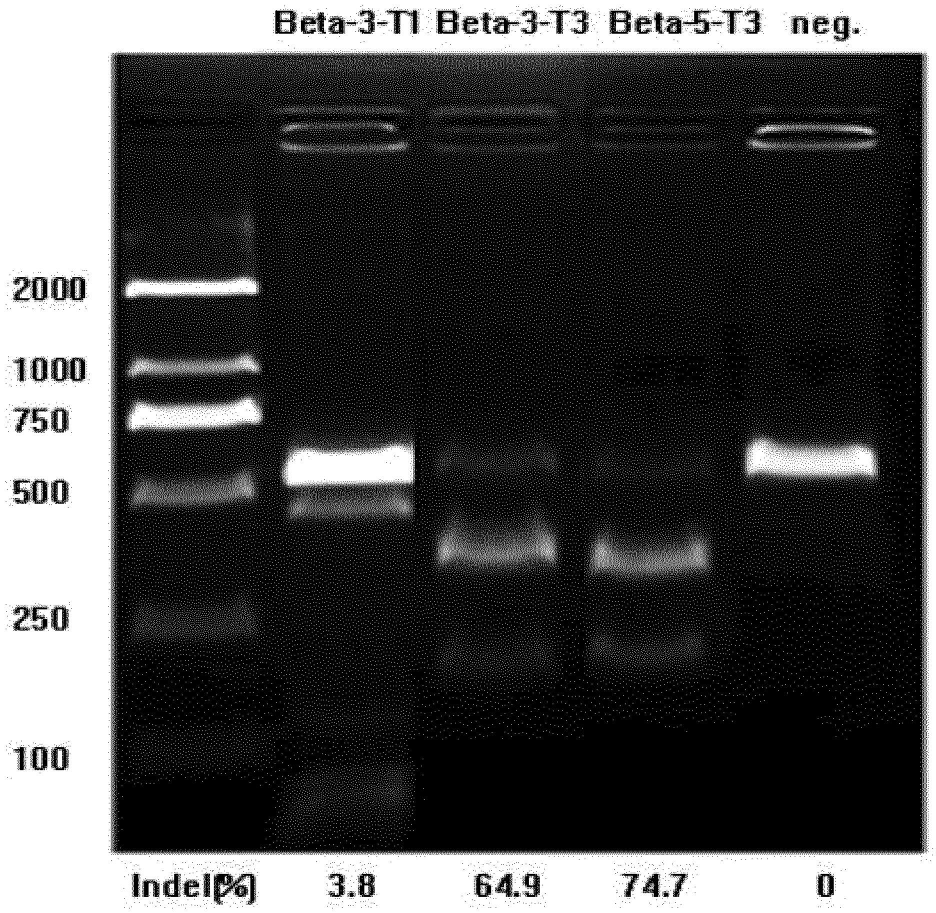
Beta-3-T1
Beta-3-T3
Beta-5-T3
neg.
2000
1000
750
500
250
100
Indel(%)
3.8
64.9
74.7
0

Fig. 10

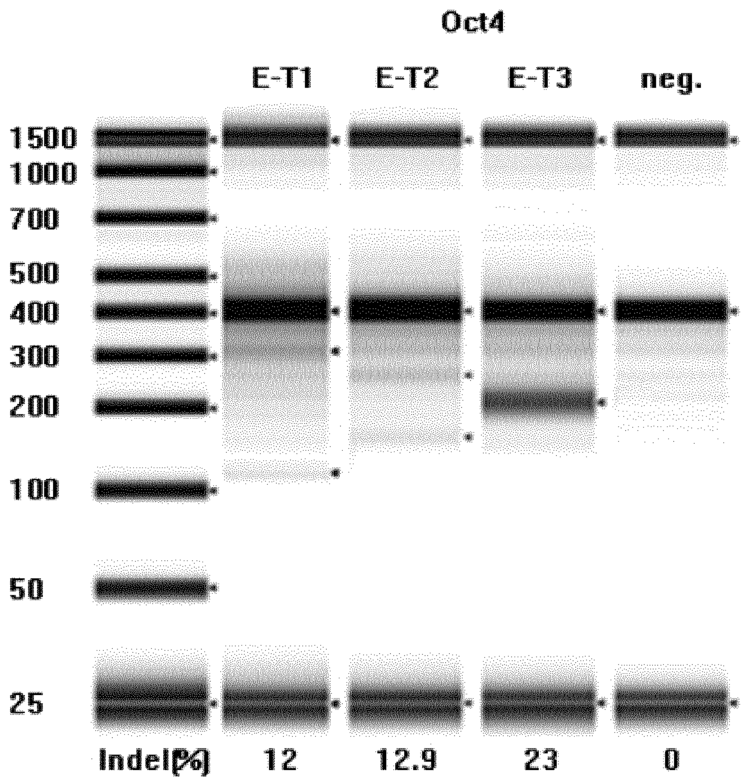
Oct4
E-T1
E-T2
E-T3
neg.
1500
1000
700
500
400
300
200
100
50
25
Indel(%)
12
12.9
23
0

Fig. 11

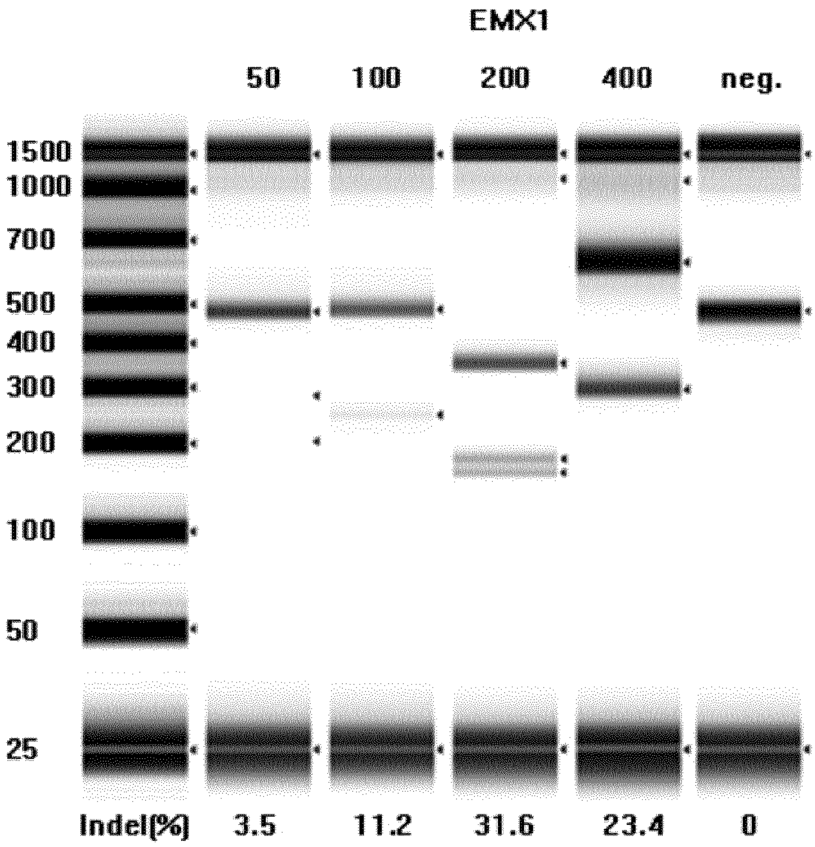
EMX1
50
100
200
400
neg.
1500
1000
700
500
400
300
200
100
50
25
Indel(%)
3.5
11.2
31.6
23.4
0

Fig. 12

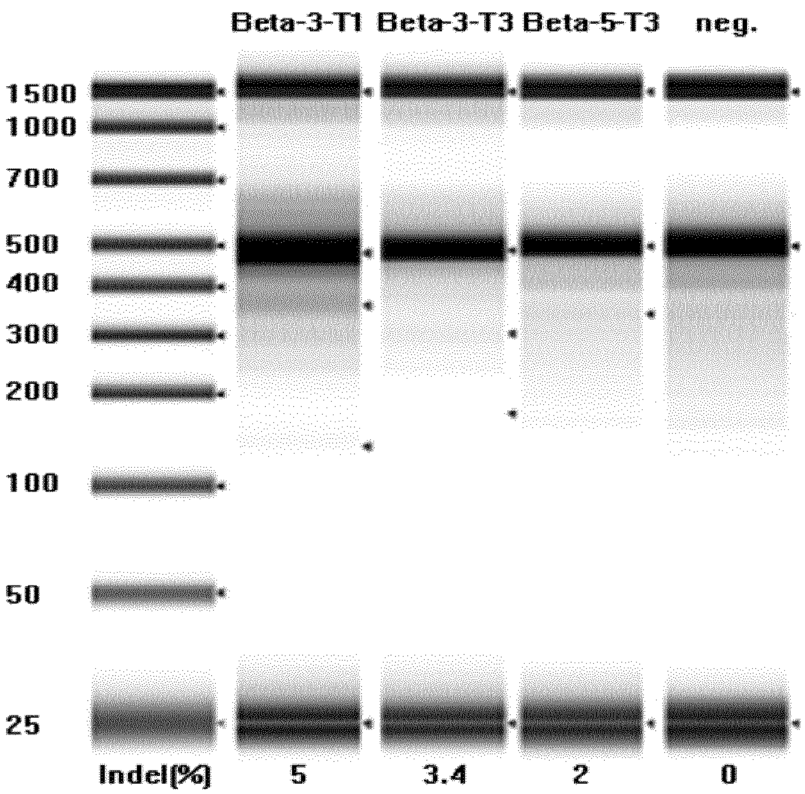
Beta-3-T1
Beta-3-T3
Beta-5-T3
neg.
1500
1000
700
500
400
300
200
100
50
25
Indel(%)
5
3.4
2
0

Fig. 13

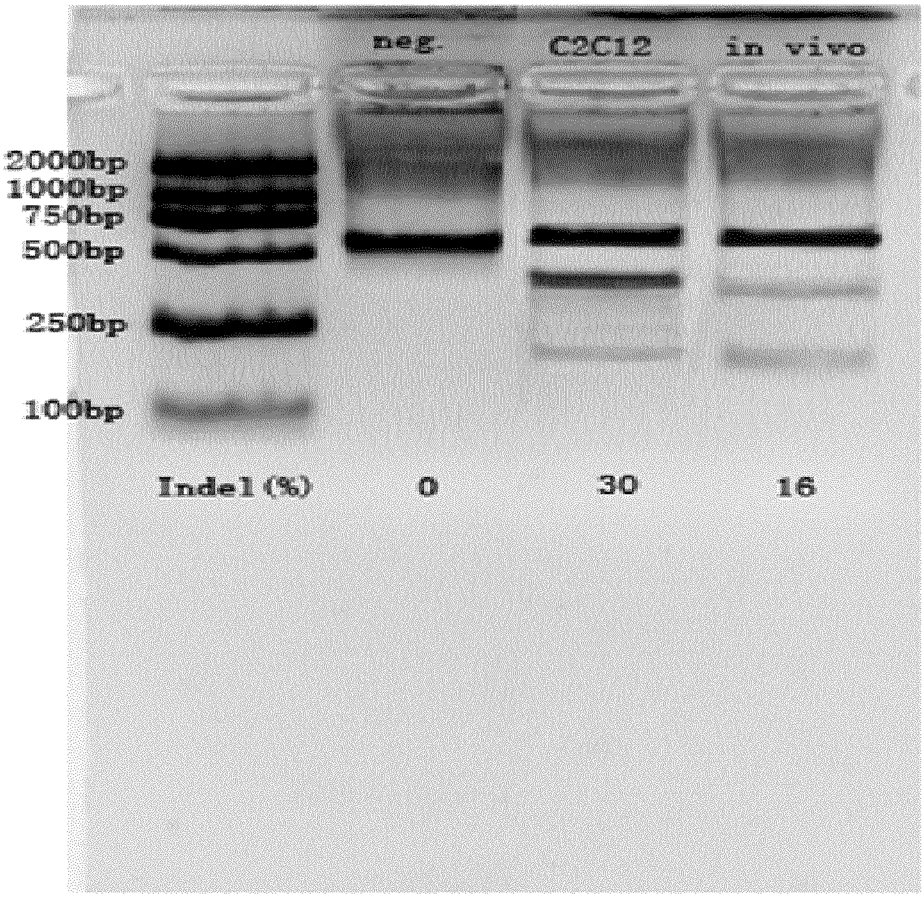
neg.
C2C12
in vivo
2000bp
1000bp
750bp
500bp
250bp
100bp
Indel(%)
0
30
16

Fig. 14

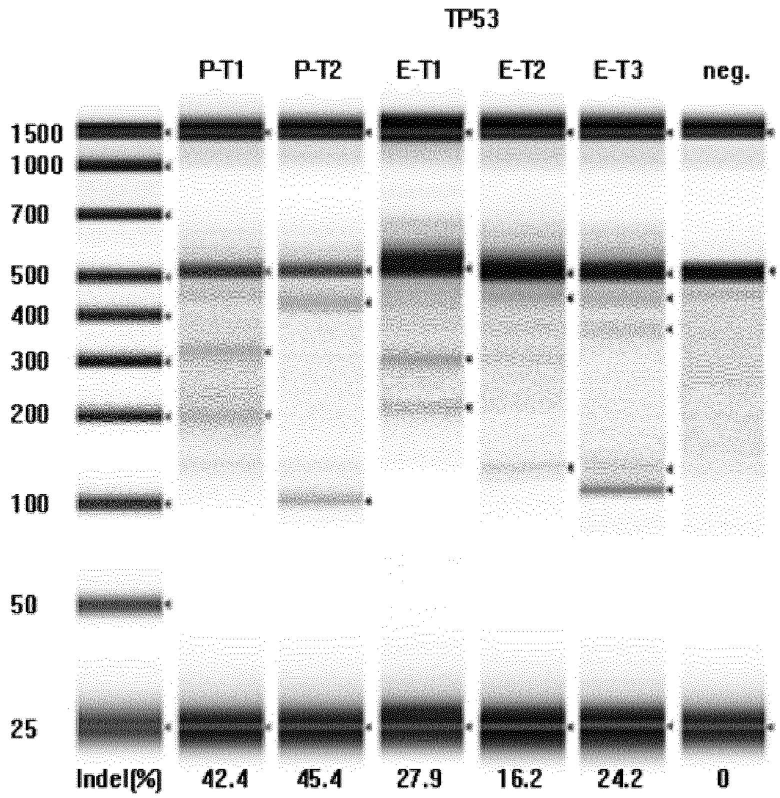
TP53
P-T1
P-T2
E-T1
E-T2
E-T3
neg.
1500
1000
700
500
400
300
200
100
50
25
Indel(%)
42.4
45.4
27.9
16.2
24.2
0

Fig. 15

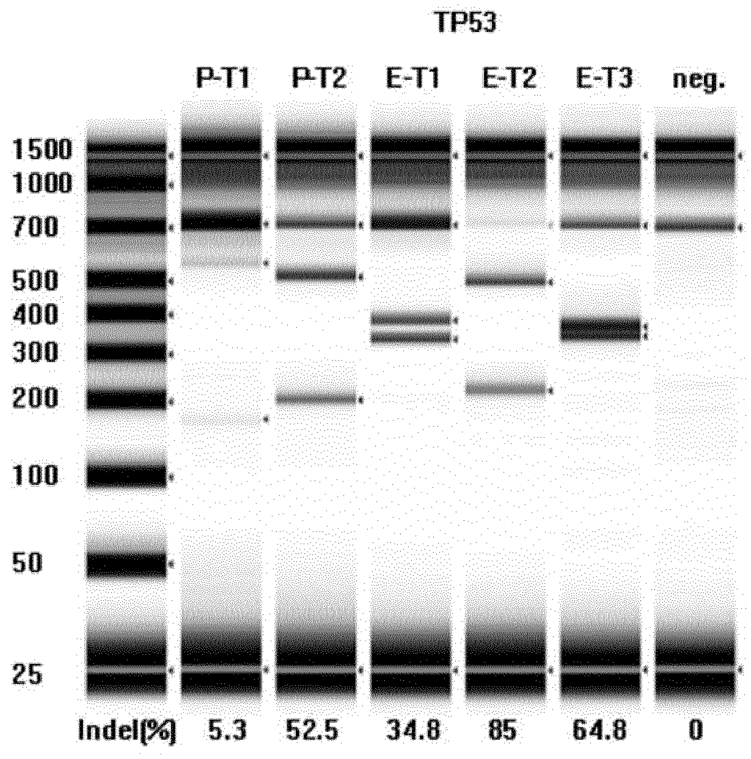
TP53
P-T1
P-T2
E-T1
E-T2
E-T3
neg.
1500
1000
700
500
400
300
200
100
50
25
Indel(%)
5.3
52.5
34.8
85
64.8
0

Fig. 16

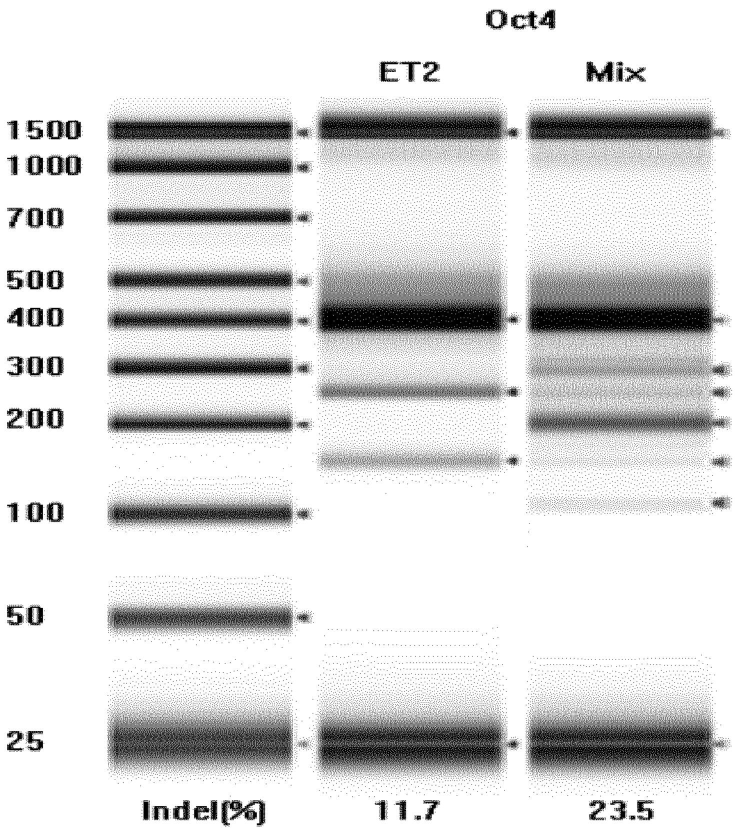
Oct4
ET2
Mix
1500
1000
700
500
400
300
200
100
50
25
Indel(%)
11.7
23.5

Fig. 17

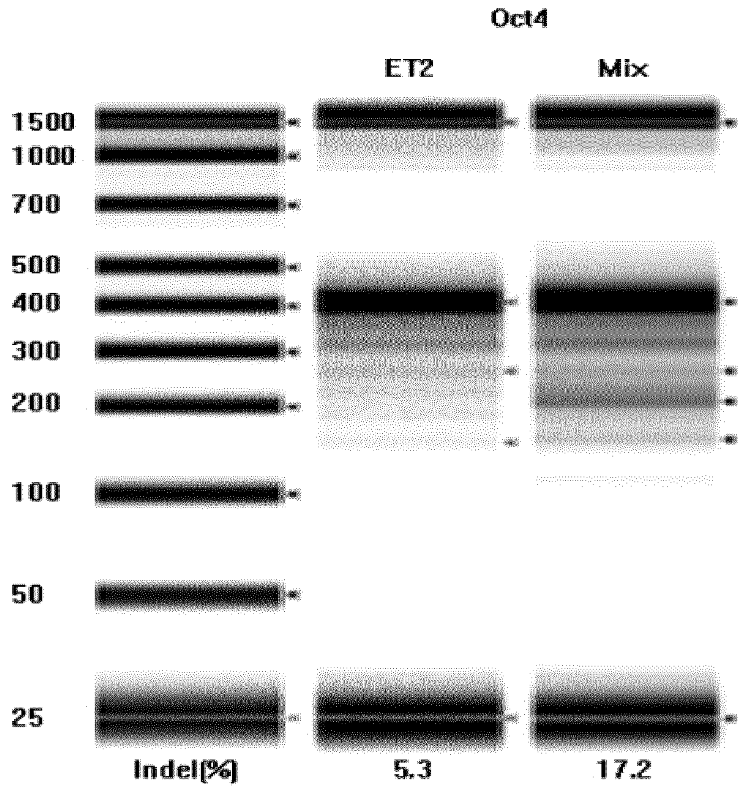
Oct4
ET2
Mix
1500
1000
700
500
400
300
200
100
50
25
Indel(%)
5.3
17.2

Fig. 18

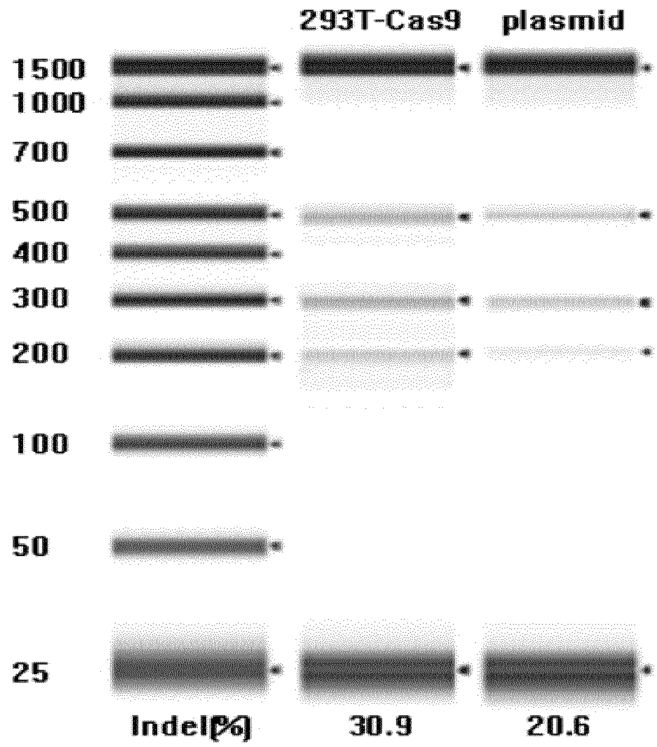
293T-Cas9
plasmid
1500
1000
700
500
400
300
200
100
50
25
Indel(%)
30.9
20.6

Fig. 19

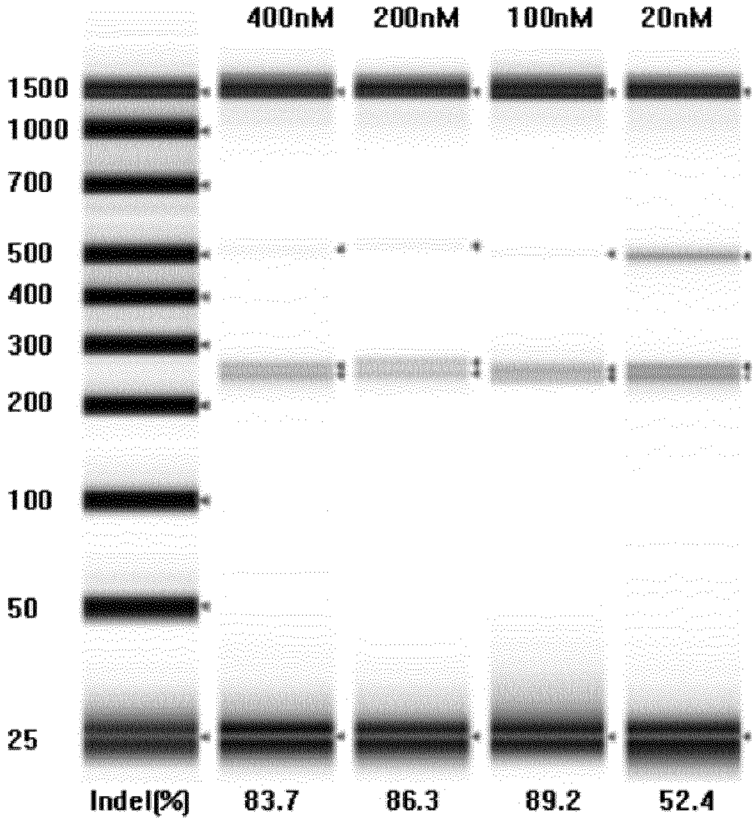
400nM
200nM
100nM
20nM
1500
1000
700
500
400
300
200
100
50
25
Indel(%)
83.7
86.3
89.2
52.4

Fig. 20

# INTERNATIONAL SEARCH REPORT

International application No.
PCT/CN2018/088105

**A. CLASSIFICATION OF SUBJECT MATTER**

C12N 15/113 (2010.01) i; C12N 15/63 (2006.01) i; C12N 9/22 (2006.01) i

According to International Patent Classification (IPC) or to both national classification and IPC

B. FIELDS SEARCHED

Minimum documentation searched (classification system followed by classification symbols)

C12N

Documentation searched other than minimum documentation to the extent that such documents are included in the fields searched

Electronic data base consulted during the international search (name of data base and, where practicable, search terms used)

CPRSABS; CNTXT; EPTXT; CNABS; JPTXT; HKABS; USTXT; WOTXT; SIPOABS; DWPI; CNKI; WANFANG DATABASE; WEB OF SCIENCE: 基因编辑, DNA 编辑, 修饰, gene edit, DNA edit, CRISPR, Cas, crRNA, tracrRNA;
NATIONAL BIO-SEQUENCE DATABASE OF CHINESE PATENT+GenBank +EMBL: sequence search for SEQ ID NOs: 1-29

C. DOCUMENTS CONSIDERED TO BE RELEVANT

| Category* | Citation of document, with indication, where appropriate, of the relevant passages | Relevant to claim No. |
|---|---|---|
| A | CN 104968784 A (TOOLGEN INC.), 07 October 2015 (07.10.2015), see entire document | 1-10 |
| A | CN 105441440 A (TOOLGEN INC.), 30 March 2016 (30.03.2016), see entire document | 1-10 |
| A | CN 106459995 A (EDITAS MEDICINE INC. et al.), 22 February 2017 (22.02.2017), see entire document | 1-10 |
| A | WO 2017040511 A1 (AGILENT TECHNOLOGIES INC.), 09 March 2017 (09.03.2017), see entire document | 1-10 |
| A | US 2015232881 A1 (GLUCKSMANN, A. et al.), 20 August 2015 (20.08.2015), see entire document | 1-10 |
| A | CN 106701810 A (JIANGNAN UNIVERSITY), 24 May 2017 (24.05.2017), see entire document | 1-10 |
| A | CN 106103706 A (THE GENERAL HOSPITAL CORPORATION), 09 November 2016 (09.11.2016), see entire document | 1-10 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 20 August 2018 | 27 August 2018 |

| Name and mailing address of the ISA | Authorized officer |
|---|---|
| State Intellectual Property Office of the P. R. China<br>No. 6, Xitucheng Road, Jimenqiao<br>Haidian District, Beijing 100088, China<br>Facsimile No. (86-10) 62019451 | WANG, Huimei<br>Telephone No. 62416241 |

Form PCT/ISA/210 (second sheet) (January 2015)

**INTERNATIONAL SEARCH REPORT**
Information on patent family members

International application No.
PCT/CN2018/088105

| Patent Documents referred in the Report | Publication Date | Patent Family | Publication Date |
|---|---|---|---|
| CN 104968784 A | 07 October 2015 | SG 11201503059X A | 29 June 2015 |
| | | KR 20180029092 A | 19 March 2018 |
| | | HK 1223125 A1 | 21 July 2017 |
| | | DE 202013012610 U1 | 24 November 2017 |
| | | US 2015322457 A1 | 12 November 2015 |
| | | US 2015344912 A1 | 03 December 2015 |
| | | AU 2013335451 A1 | 07 May 2015 |
| | | KR 20150101476 A | 03 September 2015 |
| | | EP 3346003 A1 | 11 July 2018 |
| | | BR 112015008708 A2 | 26 September 2017 |
| | | AU 2017254854 A1 | 16 November 2017 |
| | | JP 2016500003 A | 07 January 2016 |
| | | KR 20150101478 A | 03 September 2015 |
| | | AU 2013335451 B2 | 15 September 2016 |
| | | JP 2016027807 A | 25 February 2016 |
| | | KR 101706085 B1 | 14 February 2017 |
| | | AU 2015218519 A1 | 17 September 2015 |
| | | US 2015284727 A1 | 08 October 2015 |
| | | EP 2912175 A1 | 02 September 2015 |
| | | WO 2014065596 A1 | 01 May 2014 |
| | | KR 101656236 B1 | 12 September 2016 |
| | | KR 20150101477 A | 03 September 2015 |
| | | CA 2888190 A1 | 01 May 2014 |
| | | KR 101656237 B1 | 12 September 2016 |
| | | CN 105441440 A | 30 March 2016 |
| | | KR 20150101446 A | 03 September 2015 |
| | | JP 2018019698 A | 08 February 2018 |
| | | EP 2912175 A4 | 09 December 2015 |
| | | DE 202013012597 U1 | 21 November 2017 |
| | | HK 1212732 A1 | 17 June 2016 |
| CN 105441440 A | 30 March 2016 | SG 11201503059X A | 29 June 2015 |
| | | KR 20180029092 A | 19 March 2018 |
| | | HK 1223125 A1 | 21 July 2017 |
| | | DE 202013012610 U1 | 24 November 2017 |
| | | US 2015322457 A1 | 12 November 2015 |
| | | US 2015344912 A1 | 03 December 2015 |
| | | AU 2013335451 A1 | 07 May 2015 |
| | | KR 20150101476 A | 03 September 2015 |
| | | EP 3346003 A1 | 11 July 2018 |
| | | BR 112015008708 A2 | 26 September 2017 |
| | | AU 2017254854 A1 | 16 November 2017 |
| | | JP 2016500003 A | 07 January 2016 |
| | | CN 104968784 A | 07 October 2015 |
| | | KR 20150101478 A | 03 September 2015 |
| | | AU 2013335451 B2 | 15 September 2016 |
| | | JP 2016027807 A | 25 February 2016 |
| | | KR 101706085 B1 | 14 February 2017 |
| | | AU 2015218519 A1 | 17 September 2015 |
| | | US 2015284727 A1 | 08 October 2015 |
| | | EP 2912175 A1 | 02 September 2015 |

Form PCT/ISA/210 (patent family annex) (January 2015)

**INTERNATIONAL SEARCH REPORT**
Information on patent family members

International application No.
PCT/CN2018/088105

| Patent Documents referred in the Report | Publication Date | Patent Family | Publication Date |
|---|---|---|---|
| | | WO 2014065596 A1 | 01 May 2014 |
| | | KR 101656236 B1 | 12 September 2016 |
| | | KR 20150101477 A | 03 September 2015 |
| | | CA 2888190 A1 | 01 May 2014 |
| | | KR 101656237 B1 | 12 September 2016 |
| | | KR 20150101446 A | 03 September 2015 |
| | | JP 2018019698 A | 08 February 2018 |
| | | EP 2912175 A4 | 09 December 2015 |
| | | DE 202013012597 U1 | 21 November 2017 |
| | | HK 1212732 A1 | 17 June 2016 |
| CN 106459995 A | 22 February 2017 | PT 3066201 T | 04 June 2018 |
| | | CA 2930015 A1 | 14 May 2015 |
| | | EP 3066201 B1 | 07 March 2018 |
| | | WO 2015070083 A1 | 14 May 2015 |
| | | US 2015232881 A1 | 20 August 2015 |
| | | US 9834791 B2 | 05 December 2017 |
| | | EP 3066201 A1 | 14 September 2016 |
| | | ES 2670983 T3 | 04 June 2018 |
| | | US 2018127783 A1 | 10 May 2018 |
| | | DK 3066201 T3 | 06 June 2018 |
| | | AU 2014346559 A1 | 23 June 2016 |
| | | JP 2016536021 A | 24 November 2016 |
| | | KR 20160105781 A | 07 September 2016 |
| WO 2017040511 A1 | 09 March 2017 | US 2017058298 A1 | 02 March 2017 |
| | | CN 107922949 A | 17 April 2018 |
| | | KR 20180037297 A | 11 April 2018 |
| | | EP 3344771 A1 | 11 July 2018 |
| US 2015232881 A1 | 20 August 2015 | PT 3066201 T | 04 June 2018 |
| | | CA 2930015 A1 | 14 May 2015 |
| | | EP 3066201 B1 | 07 March 2018 |
| | | WO 2015070083 A1 | 14 May 2015 |
| | | US 9834791 B2 | 05 December 2017 |
| | | EP 3066201 A1 | 14 September 2016 |
| | | CN 106459995 A | 22 February 2017 |
| | | ES 2670983 T3 | 04 June 2018 |
| | | US 2018127783 A1 | 10 May 2018 |
| | | DK 3066201 T3 | 06 June 2018 |
| | | AU 2014346559 A1 | 23 June 2016 |
| | | JP 2016536021 A | 24 November 2016 |
| | | KR 20160105781 A | 07 September 2016 |
| CN 106701810 A | 24 May 2017 | None | |
| CN 106103706 A | 09 November 2016 | AU 2014370416 A1 | 07 July 2016 |
| | | EP 3090044 A4 | 05 July 2017 |
| | | CA 2935032 A1 | 02 July 2015 |
| | | KR 20160102056 A | 26 August 2016 |
| | | EP 3090044 A1 | 09 November 2016 |
| | | JP 2017505117 A | 16 February 2017 |
| | | WO 2015099850 A1 | 02 July 2015 |

Form PCT/ISA/210 (patent family annex) (January 2015)